# EUROPEAN PATENT APPLICATION

(11) **EP 4 198 052 A1**
(43) Date of publication of application: **21.06.2023**
(21) Application number: 21214728.4
(22) Date of filing: 15.12.2021
(51) Int. Cl.: C07K 14/705, C07K 14/71, C07K 14/715, C12N 5/0783

(54) **PEPTIDES AND ANTIGEN BINDING PROTEINS FOR USE IN IMMUNOTHERAPY AGAINST FIBROLAMELLAR HEPATOCELLULAR CARCINOMA (FL-HCC) AND OTHER CANCERS**

(71) Applicant: Eberhard Karls Universität Tübingen Medizinische Fakultät, 72074 Tübingen (DE); Albert-Ludwigs-Universität Freiburg, 79098 Freiburg (DE)
(72) Inventor: Walz, Juliane, 72074 Tübingen (DE); Bauer, Jens, 72070 Tübingen (DE); Maringer, Yacine, 72070 Tübingen (DE); Köhler, Natalie, 79110 Freiburg (DE); Dicks, Severin, 79117 Freiburg (DE); Zwick, Melissa, 79117 Freiburg (DE); Börries, Melanie, 79299 Wittnau (DE)
(74) Representative: Witte, Weller & Partner Patentanwälte mbB

(57) **Abstract**

The present invention relates to peptides, antigen binding proteins, nucleic acids and cells for use in immunotherapeutic methods. In particular, the present invention relates to the immunotherapy of cancer, especially of fibrolamellar hepatocellular carcinoma (FL-HCC). The present invention furthermore relates to tumor-associated T-cell peptide epitopes and recombinant T-cell receptors that can for example serve as active pharmaceutical ingredients of vaccine compositions that stimulate anti-tumor immune responses, or to stimulate T cells *ex vivo* and transfer into patients.

## Description

The present invention relates to peptides, antigen binding proteins, nucleic acids and cells for use in immunotherapeutic methods. In particular, the present invention relates to the immunotherapy of cancer, especially of fibrolamellar hepatocellular carcinoma (FL-HCC). The present invention furthermore relates to tumor-associated T-cell peptide epitopes and recombinant T-cell receptors that can for example serve as active pharmaceutical ingredients of vaccine compositions that stimulate anti-tumor immune responses, or to stimulate T cells *ex vivo* and transfer into patients.

### FIELD OF THE INVENTION

The present invention relates to the field of molecular biology, more particular to the field of molecular immunology.

### BACKGROUND OF THE INVENTION

Cancer is the second leading cause of death globally, accounting for an estimated 9.6 million deaths, or one in six deaths, in 2018. One in 5 men and one in 6 women worldwide develop cancer during their lifetime, and one in 8 men and one in 11 women die from the disease. Worldwide, the total number of people who are alive within 5 years of a cancer diagnosis, called the 5-year prevalence, is estimated to be 43.8 million. Lung, prostate, colorectal, stomach and liver cancer are the most common types of cancer in men, while breast, colorectal, lung, cervical and thyroid cancer are the most common among women.

The increasing cancer burden is due to several factors, including population growth and ageing as well as the changing prevalence of certain causes of cancer linked to social and economic development. This is particularly true in rapidly growing economies, where a shift is observed from cancers related to poverty and infections to cancers associated with lifestyles more typical of industrialized countries.

Human fibrolamellar hepatocellular carcinomas (FL-HCCs) are rare cancers accounting for less than about 5% of all liver cancers and unique in being found primarily in children to young adults without evidence of fibrosis or cirrhosis. FL-HCC ubiquitously harbors an approximately 400-kb deletion on chromosome 19 that produces an in-frame fusion of DnaJ heat shock protein family member B1 (DNAJB1) and protein kinase cAMP-activated catalytic subunit alpha (PRKACA). DNAJB1 encodes a subunit of the heat shock factor 46 (HSP40) complex, which activates the ATPase of HSP70 and serves as a molecular chaperone that can be induced by an array of environmental stresses. PRKACA encodes a catalytic subunit of protein kinase A (PKA), which resides in the cytoplasm in an inactive tetrameric complex with PKA C-b and two regulatory subunits of the PKA holoenzyme. Activation of G protein-coupled receptors leads to cAMP-dependent activation of the PKA catalytic subunits and subsequent phosphorylation of a panoply of cellular substrates. The crystal structure of the DNAJB1-PRKACA fusion protein shows that the catalytic site, regulatory subunit binding, and anchoring protein binding remain similar to those of the wild-type PRKACA.

Beyond the presence of DNAJB1-PRKACA fusions, FL-HCC tumorigenesis is poorly understood. Few, if any, other significantly recurrent mutated genes have been described and while broad copy-number alterations have been observed, they do not specifically implicate known oncogenes or tumor suppressors. Unlike liver cancer in older adults, FL-HCC is not associated with any known etiological risk factors such as alcoholism, chronic hepatitis infection, or liver flukes.

Due to lack of symptoms, until the tumor is sizable, this form of cancer is often advanced when diagnosed. Symptoms include vague abdominal pain, nausea, abdominal fullness, malaise and weight loss. They may also include a palpable liver mass. Other presentations include jaundice, ascites, fulminant liver failure, encephalopathy, gynecomastia (males only), thrombophlebitis of the lower limbs, recurrent deep vein thrombosis, anemia and hypoglycemia. While early onset and lack of chronic liver disease are suggestive of FL-HCC, classic hepatocellular carcinoma can also occur in young patients and misdiagnosis is common.

Current treatment of FL-HCC includes aggressive surgical resection. Liver resection may need to be performed more than once, since this disease has a very high recurrence rate. Due to such recurrence, periodic follow-up medical imaging (CT or MRI) is necessary. As the tumor is quite rare, there is no standard chemotherapy regimen. Radiotherapy has been used but data is limited concerning its use. The survival rate for FL-HCC largely depends on whether and to what degree the cancer has metastasized, i.e. spread to the lymph nodes or other organs. Distant spread (metastases) significantly reduces the median survival rate. Five-year survival rates vary between 40-90%.

The DNAJB1-PRKACA fusion could also be detected in multiple other gastroenterological tumor diseases, such as oncocytic pancreatic and biliary neoplasms.

T-cell-based immunotherapies, comprising immune checkpoint inhibitors (ICI), CAR-T cells, adoptive T-cell transfer, and vaccination strategies achieved a breakthrough in the treatment of malignant disease. However, these therapies, which rely on the rejection of cancer cells through recognition of tumor antigens and T cell-mediated cytotoxicity, are still only available and effective in small subsets of cancer patients and single tumor entities. One main reason in particular for the development of antigen-specific immunotherapies is the lack of suitable target structures that show natural, highfrequency, and tumor-exclusive presentation on the cell surface of tumor cells and are recognized by the immune system. Tumor antigens are represented by HLA-independent surface molecules or by HLA class I and II-presented T-cell epitopes, originating from intracellular proteins. In terms of the latter, neoepitopes, arising from tumor-specific mutations have been identified in recent years as main specificity of anti-cancer T-cell responses induced by ICIs and thus suggested as prime candidates for T-cell-based immunotherapy approaches. In line, response to ICI is correlated with high tumor somatic mutational burden and neoepitope-based immunotherapies showed first promising results in individual tumor patients. However, patient/tumor-specificity and intratumoral heterogeneity of somatic mutations as well as the limited number of somatic mutations translated, processed, and presented as HLA-restricted neoepitope on the tumor cells restrict the broad applicability of these antigens in particular in low-mutational burden entities.

Recently, fusion transcripts, whose products often represent clonal oncogenic drivers, were identified as a novel source of highly immunogenic neoepitopes. T-cell responses against such fusion protein-derived neoepitopes were detected in patients receiving ICI and correlated with treatment response.

The DNAJB1-PRKACA fusion transcript links exon 1 of the DnaJ homolog subfamily B member 1 gene (DNAJB1) to the cAMP-dependent protein kinase catalytic subunit alpha gene (PRKACA). In FL-HCC the DNAJB1-PRKACA fusion transcript is detectable in 100% of patients and was identified as the oncogenic driver in tumor pathogenesis, indicating expression of the fusion transcript in all tumor cells.

Immunotherapeutic peptide-based approaches against FL-HCC are disclosed in WO 2020/257575 and WO 2021/138582.

None of the approaches, however, ever reached broad clinical application and efficacy, let alone approval. This may have its reason in the relative unsuitability of the described peptides to elicit FL-HCC-specific immune responses in the patients.

It is, therefore, an object underlying the invention to provide tumor-associated T-cell peptide epitopes, which can be used to develop improved medicaments and methods for the diagnosis, prophylaxis and treatment of cancer, in particular of cancer with a fusion of DnaJ heat shock protein family member B1 (*DNAJB1*) and protein kinase cAMP-activated catalytic subunit alpha (*PRKACA*) (DNAJB1-PRKACA), such as fibrolamellar hepatocellular carcinoma (FL-HCC).

The present invention satisfies these and other needs.

### SUMMARY OF THE INVENTION

The present invention provides a peptide comprising an amino acid sequence selected from the group consisting of SEQ ID NO: 1 to SEQ ID NO: 5, and variant sequences thereof which are at least 88% homologous to SEQ ID NO: 1 to SEQ ID NO: 5, and wherein said variant sequences bind to molecule(s) of the major histocompatibility complex (MHC) and/or induce T-cells cross-reacting with said variant sequences; and a pharmaceutical acceptable salt thereof, wherein said peptide is not a full-length polypeptide.

The inventors have identified and characterized the peptides according to the invention as neoantigens derived from the DNAJB1-PRKACA fusion transcript, which is the oncogenic driver in fibrolamellar hepatocellular carcinoma (FL-HCC) and many other gastroenterological tumor diseases. The peptides with SEQ ID NO: 1 and/or 2 have a universal MHC type (human: HLA allotype class II) and are, therefore, suitable for the administration to any subject or human being, respectively. The peptides with any of SEQ ID NOs: 3 - 5 are especially suitable for an administration to a subject with an appropriate MHC type (human: SEQ ID NO: 3 = HLA allotype A*68:02; SEQ ID NO: 4 = HLA allotype C*04:01 ;C*05:01; SEQ ID NO: 5 = HLA allotype A*24:02).

The inventors were able to demonstrate that the neoantigens according to the invention induce multifunctional cytotoxic CD8⁺ and T-helper (Th) 1 CD4⁺ T cells, which is required for effective anti-cancer therapy. Their natural processing and presentation in DNAJB1-PRKACA expressing tumor cells was proven by mass spectrometry-based immunopeptidome analysis. Within the framework of an individual healing experiment it could also be shown by the inventors that vaccination with the peptides according to the invention of a FL-HCC patient, suffering from recurrent disease relapses, induce multifunctional CD4⁺ T cells, with an activated Th1 phenotype and high TCR clonality. Vaccine-induced DNAJB1-PRKACA-specific T-cell responses persist over time and are accompanied by relapse-free survival of the patient more than one year post vaccination. The peptides according to the invention are, therefore, suitable active agents of a pharmaceutical composition, especially a vaccine, for the treatment and/or prophylaxis of cancer, such as FL-HCC.

The following table shows the peptides according to the present invention, the HLA allotypes, and their respective SEQ ID NOs.

**Table 1: Peptides according to the invention**

| Sequence | HLA allotype | SEQ ID NO: |
|---|---|---|
| KREIFDRYGEEVKEFLAKAKED | Class II | 1 |
| EVKEFLAKAKEDFLKK | Class II | 2 |
| EIFDRYGEEV | A*68:02 | 3 |
| IFDRYGEEV | C*04:01;C*05:01 | 4 |
| RYGEEVKEF | A*24:02 | 5 |

In the event of discrepancies between the sequences specified in Table 1 and those specified in the sequence listing, the information in the sequence listing takes precedence and applies.

The term "peptide" is used herein to designate a series of amino acid residues, connected one to the other typically by peptide bonds between the alpha-amino and carbonyl groups of the adjacent amino acids. The peptides are preferably between 7 and 12 amino acids in length, further preferably between 8 and 11, but can be as long as 5, 6, 7, 8, 9, 10, 11, 12, 13, 14, 15, 16, 17, 18, 19, 20, 21, 22, 23 or longer.

Furthermore, the term "peptide" shall include salts of a series of amino acid residues, connected one to the other typically by peptide bonds between the alpha-amino and carbonyl groups of the adjacent amino acids. Preferably, the salts are pharmaceutical acceptable salts of the peptides, such as, for example, the chloride or acetate (trifluoroacetate) salts. It has to be noted that the salts of the peptides according to the present invention differ substantially from the peptides in their state(s) *in vivo,* as the peptides are not salts *in vivo.*

The term "peptide" shall also include "oligopeptide". The term "oligopeptide" is used herein to designate a series of amino acid residues, connected one to the other typically by peptide bonds between the alpha-amino and carbonyl groups of the adjacent amino acids. The length of the oligopeptide is not critical to the invention, as long as the correct epitope or epitopes are maintained therein. The oligopeptides are typically less than about 30 amino acid residues in length, and greater than about 15 amino acids in length.

The term "peptide" shall also include "polypeptide". The term "polypeptide" designates a series of amino acid residues, connected one to the other typically by peptide bonds between the alpha-amino and carbonyl groups of the adjacent amino acids. The length of the polypeptide is not critical to the invention as long as the correct epitopes are maintained. In contrast to the terms peptide or oligopeptide, the term polypeptide is meant to refer to molecules containing more than about 30 amino acid residues.

The "major histocompatibility complex" (MHC) is a set of cell surface proteins essential for the acquired immune system to recognize foreign molecules in vertebrates, which in turn determines histocompatibility. The main function of MHC molecules is to bind to antigens derived from pathogens and display them on the cell surface for recognition by the appropriate T cells. The human MHC is also called the HLA (human leukocyte antigen) complex (or just "HLA"). The MHC gene family is divided into three subgroups: class I, class II, and class III. Complexes of peptide and MHC class I are recognized by CD8-positive T cells bearing the appropriate T cell receptor (TCR), whereas complexes of peptide and MHC class II molecules are recognized by CD4-positive-helper-T cells bearing the appropriate TCR. Since both CD8 and CD4 dependent responses contribute jointly and synergistically to the anti-viral effect, the identification and characterization of viral antigens and corresponding T cell receptors is important in the development of viral immunotherapies such as vaccines and cell therapies. The HLA-A gene is located on the short arm of chromosome 6 and encodes the larger, α-chain, constituent of HLA-A. Variation of HLA-A α-chain is key to HLA function. This variation promotes genetic diversity in the population. Since each HLA has a different affinity for peptides of certain structures, greater variety of HLAs means greater variety of antigens to be 'presented' on the cell surface.

By a "variant" of the given amino acid sequence the inventors mean that the side chains of, for example, one or two of the amino acid residues are altered (for example by replacing them with the side chain of another naturally occurring amino acid residue or some other side chain) such that the peptide is still able to bind to an MHC molecule in substantially the same way as a peptide consisting of the given amino acid sequence in consisting of SEQ ID NO: 1 to SEQ ID NO: 5. For example, a peptide may be modified so that it at least maintains, if not improves, the ability to interact with and bind to the binding groove of a suitable MHC molecule, such as HLA-A*02, and in that way, it at least maintains, if not improves, the ability to bind to the TCR of activated T cells.

The original (unmodified) peptides as disclosed herein can be modified by the substitution of one or more residues at different, possibly selective, sites within the peptide chain, if not otherwise stated. Preferably those substitutions are located at the end of the amino acid chain. Such substitutions may be of a conservative nature, for example, where one amino acid is replaced by an amino acid of similar structure and characteristics, such as where a hydrophobic amino acid is replaced by another hydrophobic amino acid. Even more conservative would be replacement of amino acids of the same or similar size and chemical nature, such as where leucine is replaced by isoleucine. In studies of sequence variations in families of naturally occurring homologous proteins, certain amino acid substitutions are more often tolerated than others, and these are often show correlation with similarities in size, charge, polarity, and hydrophobicity between the original amino acid and its replacement, and such is the basis for defining "conservative substitutions". Conservative substitutions are herein defined as exchanges within one of the following five groups: Group 1-small aliphatic, nonpolar or slightly polar residues (Ala, Ser, Thr, Pro, GIY); Group 2-polar, negatively charged residues and their amides (Asp, Asn, Glu, Gin); Group 3-polar, positively charged residues (His, Arg, Lys); Group 4-large, aliphatic, nonpolar residues (Met, Leu, Ile, Val, Cys); and Group 5-large, aromatic residues (Phe, Tyr, Trp). Less conservative substitutions might involve the replacement of one amino acid by another that has similar characteristics but is somewhat different in size, such as replacement of an alanine by an isoleucine residue. Highly non-conservative replacements might involve substituting an acidic amino acid for one that is polar, or even for one that is basic in character. Such "radical" substitutions cannot, however, be dismissed as potentially ineffective since chemical effects are not totally predictable and radical substitutions might well give rise to serendipitous effects not otherwise predictable from simple chemical principles. Of course, such substitutions may involve structures other than the common L-amino acids. Thus, D-amino acids might be substituted for the L-amino acids commonly found in the antigenic peptides of the invention and yet still be encompassed by the disclosure herein. In addition, non-standard amino acids (i.e., other than the common naturally occurring proteinogenic amino acids) may also be used for substitution purposes to produce immunogens and immunogenic polypeptides according to the present invention.

If substitutions at more than one position are found to result in a peptide with substantially equivalent or greater antigenic activity as defined below, then combinations of those substitutions will be tested to determine if the combined substitutions result in additive or synergistic effects on the antigenicity of the peptide. At most, no more than 4 positions within the peptide would be simultaneously substituted.

The amino acid residues that do not substantially contribute to interactions with the T cell receptor can be modified by replacement with other amino acids whose incorporation do not substantially affect T cell reactivity and does not eliminate binding to the relevant MHC.

Longer (elongated) peptides may also be suitable. It is possible that MHC class I epitopes, although usually between 8 and 11 amino acids long, are generated by peptide processing from longer peptides or proteins that include the actual epitope. It is preferred that the residues that flank the actual epitope are residues that do not substantially affect proteolytic cleavage necessary to expose the actual epitope during processing.

The peptides of the invention can be elongated by up to four amino acids, that is 1, 2, 3 or 4 amino acids can be added to either end in any combination between 4:0 and 0:4. Combinations of the elongations according to the invention can be found in Table 2.

**Table 2: Combinations of the elongations of peptides of the invention**

| C-terminus | N-terminus |
|---|---|
| 4 | 0 |
| 3 | 0 or 1 |
| 2 | 0 or 1 or 2 |
| 1 | 0 or 1 or 2 or 3 |
| 0 | 0 or 1 or 2 or 3 or 4 |

| N-terminus | C-terminus |
|---|---|
| 4 | 0 |
| 3 | 0 or 1 |
| 2 | 0 or 1 or 2 |
| 1 | 0 or 1 or 2 or 3 |
| 0 | 0 or 1 or 2 or 3 or 4 |

The amino acids for the elongation/extension can be from the peptides of the original sequence of the protein or any other amino acid(s). The elongation can be used to enhance the stability or solubility of the peptides.

Thus, the epitopes of the present invention may be identical to naturally occurring tumor-associated or tumor-specific epitopes or may include epitopes that differ by no more than four residues from the reference peptide, as long as they have substantially identical antigenic activity.

In an alternative embodiment, the peptide is elongated on either or both sides by more than 4 amino acids, preferably to a total length of up to 30 amino acids. This may lead to MHC class II binding peptides. Binding to MHC class II can be tested by methods known in the art.

Accordingly, the present invention provides peptides and variants of MHC class I epitopes, wherein the peptide or variant has an overall length of between 8 and 100, preferably between 8 and 30, and most preferred between 8 and 14, namely 8, 9, 10, 11, 12, 13, 14 amino acids, in case of the elongated class II binding peptides the length can also be 15, 16, 17, 18, 19, 20, 21 or 22 or 23 amino acids.

Of course, the peptide or variant according to the present invention will have the ability to bind to a molecule of the human major histocompatibility complex (MHC) class I or II. Binding of a peptide or a variant to a MHC complex may be tested by methods known in the art.

Preferably, when the T cells specific for a peptide according to the present invention are tested against the substituted peptides, the peptide concentration at which the substituted peptides achieve half the maximal increase in lysis relative to background is no more than about 1 mM, preferably no more than about 1 µM, more preferably no more than about 1 nM, and still more preferably no more than about 100 pM, and most preferably no more than about 10 pM. It is also preferred that the substituted peptide be recognized by T cells from more than one individual, at least two, and more preferably three individuals.

A person skilled in the art will be able to assess, whether T cells induced by a variant of a specific peptide will be able to cross-react with the peptide itself (Appay et al., 2006; Colombetti et al., Eur. J. Immunol. 36: 1805-1814 (2006); Fong et al., Proc. Natl. Acad. Sci. U.S.A. 98: 8809-8814 (2001); Zaremba et al., Cancer Res. 57: 4570-4577 (1997)).

These T cells can subsequently cross-react with cells and kill cells that express a polypeptide that contains the natural amino acid sequence of the cognate peptide as defined in the aspects of the invention. As can be derived from the scientific literature and databases (Rammensee et al., Immunogenetics 50: 213-219 (1999); Godkin et al., Int. Immunol 9: 905-911 (1997)), certain positions of HLA binding peptides are typically anchor residues forming a core sequence fitting to the binding motif of the HLA receptor, which is defined by polar, electrophysical, hydrophobic and spatial properties of the polypeptide chains constituting the binding groove. Thus, one skilled in the art would be able to modify the amino acid sequences set forth in SEQ ID NO: 1 to SEQ ID NO: 5, by maintaining the known anchor residues, and would be able to determine whether such variants maintain the ability to bind MHC class I or II molecules. The variants of the present invention retain the ability to bind to the TCR of activated T cells, which can subsequently cross-react with and kill cells that express a polypeptide containing the natural amino acid sequence of the cognate peptide as defined in the aspects of the invention.

In the present invention, the term "homologous" refers to the degree of identity between sequences of two amino acid sequences, i.e. peptide or polypeptide sequences. The aforementioned "homology" is determined by comparing two sequences aligned under optimal conditions over the sequences to be compared. Such a sequence homology can be calculated by creating an alignment using, for example, the ClustalW algorithm. Commonly available sequence analysis software, more specifically, Vector NTI, GENETYX or other tools are provided by public databases.

"Percent homology" or "percent homologous" in turn, when referring to a sequence, means that a sequence is compared to a claimed or described sequence after alignment of the sequence to be compared (the "Compared Sequence") with the described or claimed sequence (the "Reference Sequence"). The percent homology (synonym: percent identity) is then determined according to the following formula: percent identity = 100 [1 -(C/R)] wherein C is the number of differences between the Reference Sequence and the Compared Sequence over the length of alignment between the Reference Sequence and the Compared Sequence, wherein
(i) each base or amino acid in the Reference Sequence that does not have a corresponding aligned base or amino acid in the Compared Sequence and
(ii) each gap in the Reference Sequence and
(iii) each aligned base or amino acid in the Reference Sequence that is different from an aligned base or amino acid in the Compared Sequence, constitutes a difference and
(iv) the alignment has to start at position 1 of the aligned sequences;
and R is the number of bases or amino acids in the Reference Sequence over the length of the alignment with the Compared Sequence with any gap created in the Reference Sequence also being counted as a base or amino acid.

If an alignment exists between the Compared Sequence and the Reference Sequence for which the percent identity as calculated above is about equal to or greater than a specified minimum Percent Identity then the Compared Sequence has the specified minimum percent identity to the Reference Sequence even though alignments may exist in which the herein above calculated percent identity is less than the specified percent identity.

Methods for comparing the identity/homology of two or more sequences are known in the art. For example, the "needle" program, which uses the Needleman-Wunsch global alignment algorithm (Needleman and Wunsch, 1970 J. Mol. Biol. 48:443-453) to find the optimum alignment (including gaps) of two sequences when considering their entire length may be used. The needle program is for example available on 30 the World Wide Web site and is further described in the following publication (EMBOSS: The European Molecular Biology Open Software Suite (2000) Rice, P. Longden, I. and Bleasby, A. Trends in Genetics 16, (6) pp. 276-277). The percentage of identity between two polypeptides, in accordance with the disclosure, is calculated using the EMBOSS: needle (global) program with a "Gap Open" parameter equal to 10.0, a "Gap Extend" parameter equal 35 to 0.5, and a Blosum62 matrix.

A variant sequence which is "at least 88% homologous" refers to a sequence having, over 20 its entire length, at least about 88%, or more, in particular at least about 90%, at least about 91%, at least about 92%, at least about 93%, at least about 94%, at least about 95%, at least about 96%, at least about 97%, at least about 98%, at least about 99% or 100% sequence identity with the entire length of a reference sequence.

Proteins with an amino acid sequence "at least about 88%, at least about 90%, at least about 91%, at least about 92%, at least about 93%, at least about 94%, at least about 95%, at least about 96%, at least about 97%, at least about 98%, at least about 99% or 100% identical" to a reference sequence may comprise amino acid mutations such as deletions, insertions and/or substitutions compared to the reference sequence.

The "major histocompatibility complex" (MHC) is a set of cell surface proteins essential for the acquired immune system to recognize foreign molecules in vertebrates, which in turn determines histocompatibility. The main function of MHC molecules is to bind to antigens derived from pathogens and display them on the cell surface for recognition by the appropriate T cells. The human MHC is also called the HLA (human leukocyte antigen) complex (or just "HLA"). The MHC gene family is divided into three subgroups: class I, class II, and class III. Complexes of peptide and MHC class I are recognized by CD8-positive T cells bearing the appropriate T cell receptor (TCR), whereas complexes of peptide and MHC class II molecules are recognized by CD4-positive-helper-T cells bearing the appropriate TCR. Since both CD8 and CD4 dependent responses contribute jointly and synergistically to the anti-viral effect, the identification and characterization of viral antigens and corresponding T cell receptors is important in the development of viral immunotherapies such as vaccines and cell therapies. The HLA-A gene is located on the short arm of chromosome 6 and encodes the larger, α-chain, constituent of HLA-A. Variation of HLA-A α-chain is key to HLA function. This variation promotes genetic diversity in the population. Since each HLA has a different affinity for peptides of certain structures, greater variety of HLAs means greater variety of antigens to be 'presented' on the cell surface.

"Pharmaceutically" or "pharmaceutically acceptable" refers to molecular entities and compositions (or salts) that do not produce an adverse, allergic or other untoward reaction when administered to a mammal, especially a human, as appropriate and which do not interfere with the activity of the pharmaceutically active ingredient.

According to the invention "full-length polypeptide" refers to the source proteins from which the peptides are derived, e.g. the DNAJB1-PRKACA fusion transcript. Full-length polypeptides are also referred to as the source genes/proteins from which the peptides are derived. The source proteins or full-length polypeptides may or may not be highly over-expressed in cancer compared with normal tissues. "Normal tissues" in relation to this invention shall mean either healthy peripheral blood mononuclear cells (PBMC) cells or other normal tissue cells, demonstrating a high degree of tumor association of the source genes. Moreover, the peptides themselves are presented on tumor tissue. "Tumor tissue" in relation to this invention shall mean a sample from a patient suffering from cancer, such as fibrolamellar hepatocellular carcinoma (FL-HCC) or other gastroenterological cancers.

In an embodiment of the invention the peptide or nucleic acid is isolated. The term "isolated" means that the material is removed from its original environment (e.g., the natural environment, if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The peptides and/or nucleotides disclosed in accordance with the present invention may also be in "purified" form. The term "purified" does not require absolute purity; rather, it is intended as a relative definition, and can include preparations that are highly purified or preparations that are only partially purified, as those terms are understood by those of skill in the relevant art. For example, individual clones isolated from a cDNA library have been conventionally purified to electrophoretic homogeneity. Purification of starting material or natural material to at least one order of magnitude, preferably two or three orders, and more preferably four or five orders of magnitude is expressly contemplated. Furthermore, a claimed polypeptide which has a purity of preferably 99.999%, or at least 99.99% or 99.9%; and even desirably 99% by weight or greater is expressly encompassed.

The peptides and nucleic acids according to the invention may be in "enriched form". As used herein, the term "enriched" means that the concentration of the material is at least about 2, 5, 10, 100, or 1000 times its natural concentration (for example), advantageously 0.01%, by weight, preferably at least about 0.1% by weight. Enriched preparations of about 0.5%, 1%, 5%, 10%, and 20% by weight are also contemplated. The sequences, constructs, vectors, clones, and other materials comprising the present invention can advantageously be in enriched or isolated form.

The problem underlying the invention is herewith completely solved.

In a particularly preferred embodiment of the invention the peptide consists or consists essentially of an amino acid sequence according to SEQ ID NO: 1 to SEQ ID NO: 5.

"Consisting essentially of" shall mean that a peptide according to the present invention, in addition to the sequence according to any of SEQ ID NO: 1 to SEQ ID NO: 5 or a variant thereof contains additional N- and/or C-terminally located stretches of amino acids that are not necessarily forming part of the peptide that functions as an epitope for MHC molecules epitope.

Nevertheless, these stretches can be important to provide an efficient introduction of the peptide according to the present invention into the cells. In one embodiment of the present invention, the peptide is part of a fusion protein which comprises, for example, N- or C-terminal amino acids. In other fusions, the peptides of the present invention can be fused to an antibody as described herein, or a functional part thereof, in particular into a sequence of an antibody, so as to be specifically targeted by said antibody, or, for example, to or into an antibody that is specific for dendritic cells as described herein.

In addition, the peptide or variant may be modified further to improve stability and/or binding to MHC molecules in order to elicit a stronger immune response. Methods for such an optimization of a peptide sequence are well known in the art and include, for example, the introduction of reverse peptide bonds or non-peptide bonds.

In an embodiment of the invention said peptide has the ability to bind to an MHC class I or II molecule, and wherein said peptide, when bound to said MHC, is capable of being recognized by CD4 and/or CD8 T cells.

This measure has the advantage that the capability of the peptide according to the invention to induce an immune response, in particular a T cell response, is ensured.

In another embodiment of the invention the amino acid sequence of the peptide or variant thereof according to the invention comprises a continuous stretch of amino acids of any one of SEQ ID NO: 1 to SEQ ID NO: 5.

This measure has the advantage that the peptide or variant thereof according to the invention comprises all amino acids which are predicted as being involved in the induction of an immune response. The therapeutic efficacy is herewith further improved.

Another subject-matter of the present invention relates to an antibody, in particular a soluble or membrane-bound antibody, preferably a monoclonal antibody or fragment thereof, which specifically recognizes the peptide or variant thereof according to the invention, preferably when bound to an MHC molecule.

The term "antibody" or "antibodies" is used herein in a broad sense and includes both polyclonal and monoclonal antibodies. In addition to intact or "full" immunoglobulin molecules, also included in the term "antibodies" are fragments (e.g. CDRs, Fv, Fab and Fc fragments) or polymers of those immunoglobulin molecules and humanized versions of immunoglobulin molecules, as long as they exhibit any of the desired properties, i.e. specifically recognize the peptide or variant thereof according to the invention. Whenever possible, the antibodies of the invention may be purchased from commercial sources. The antibodies of the invention may also be generated using well-known methods.

The features, characteristics, advantages and embodiments disclosed for the peptide according to the invention apply to the antibody and fragment thereof correspondingly.

Another subject-matter of the invention relates to a T-cell receptor, preferably soluble or membrane-bound, or a fragment thereof, that is reactive with an HLA ligand, wherein said ligand is the peptide or variant thereof according to the invention, preferably when bound to an MHC molecule.

The term "T-cell receptor" (abbreviated TCR) according to the invention refers to a heterodimeric molecule comprising an alpha polypeptide chain (alpha chain) and a beta polypeptide chain (beta chain), wherein the heterodimeric receptor is capable of binding to a peptide antigen presented by an HLA molecule. The term also includes so-called gamma/delta TCRs.

The features, characteristics, advantages and embodiments disclosed for the peptide and antibody or fragment thereof according to the invention apply to the T-cell receptor correspondingly.

Another subject-matter of the invention relates to an antigen binding protein which specifically binds at least one DNAJB1-PRKACA antigenic peptide in complex with a major histocompatibility complex (MHC) molecule, wherein the antigen-binding protein comprises a first polypeptide chain which comprises a first variable domain that comprises at least one complementary determining region (CDR), CDR3α, and a second polypeptide chain which comprises at a second variable domain that comprises at least one CDR, CDR3β, wherein
a) CDR3α comprises or consists of the amino acid sequence SEQ ID NO: 6, CDR3β comprises or consists of the amino acid sequence SEQ ID NO: 36, and wherein the DNAJB1-PRKACA antigenic peptide comprises or consists of SEQ ID NO: 1; or
b) CDR3α comprises or consists of the amino acid sequence SEQ ID NO: 7, CDR3β comprises or consists of the amino acid sequence SEQ ID NO: 37, and wherein the DNAJB1-PRKACA antigenic peptide comprises or consists of SEQ ID NO: 1; or
c) CDR3α comprises or consists of the amino acid sequence SEQ ID NO: 8, CDR3β comprises or consists of the amino acid sequence SEQ ID NO: 38, and wherein the DNAJB1-PRKACA antigenic peptide comprises or consists of SEQ ID NO: 1; or
d) CDR3α comprises or consists of the amino acid sequence SEQ ID NO: 9, CDR3β comprises or consists of the amino acid sequence SEQ ID NO: 39, and wherein the DNAJB1-PRKACA antigenic peptide comprises or consists of SEQ ID NO: 1; or
e) CDR3α comprises or consists of the amino acid sequence SEQ ID NO: 10, CDR3β comprises or consists of the amino acid sequence SEQ ID NO: 40, and wherein the DNAJB1-PRKACA antigenic peptide comprises or consists of SEQ ID NO: 1; or
f) CDR3α comprises or consists of the amino acid sequence SEQ ID NO: 11, CDR3β comprises or consists of the amino acid sequence SEQ ID NO: 41, and wherein the DNAJB1-PRKACA antigenic peptide comprises or consists of SEQ ID NO: 1; or
g) CDR3α comprises or consists of the amino acid sequence SEQ ID NO: 12, CDR3β comprises or consists of the amino acid sequence SEQ ID NO: 42, and wherein the DNAJB1-PRKACA antigenic peptide comprises or consists of SEQ ID NO: 1; or
h) CDR3α comprises or consists of the amino acid sequence SEQ ID NO: 13, CDR3β comprises or consists of the amino acid sequence SEQ ID NO: 43, and wherein the DNAJB1-PRKACA antigenic peptide comprises or consists of SEQ ID NO: 1; or
i) CDR3α comprises or consists of the amino acid sequence SEQ ID NO: 14, CDR3β comprises or consists of the amino acid sequence SEQ ID NO: 44, and wherein the DNAJB1-PRKACA antigenic peptide comprises or consists of SEQ ID NO: 1; or
j) CDR3α comprises or consists of the amino acid sequence SEQ ID NO: 15, CDR3β comprises or consists of the amino acid sequence SEQ ID NO: 45, and wherein the DNAJB1-PRKACA antigenic peptide comprises or consists of SEQ ID NO: 1; or
k) CDR3α comprises or consists of the amino acid sequence SEQ ID NO: 32, CDR3β comprises or consists of the amino acid sequence SEQ ID NO: 45, and wherein the DNAJB1-PRKACA antigenic peptide comprises or consists of SEQ ID NO: 1; or
l) CDR3α comprises or consists of the amino acid sequence SEQ ID NO: 16, CDR3β comprises or consists of the amino acid sequence SEQ ID NO: 46, and wherein the DNAJB1-PRKACA antigenic peptide comprises or consists of SEQ ID NO: 1; or
m) CDR3α comprises or consists of the amino acid sequence SEQ ID NO: 17, CDR3β comprises or consists of the amino acid sequence SEQ ID NO: 47, and wherein the DNAJB1-PRKACA antigenic peptide comprises or consists of SEQ ID NO: 1; or
n) CDR3α comprises or consists of the amino acid sequence SEQ ID NO: 33, CDR3β comprises or consists of the amino acid sequence SEQ ID NO: 47, and wherein the DNAJB1-PRKACA antigenic peptide comprises or consists of SEQ ID NO: 1; or
o) CDR3α comprises or consists of the amino acid sequence SEQ ID NO: 18, CDR3β comprises or consists of the amino acid sequence SEQ ID NO: 48, and wherein the DNAJB1-PRKACA antigenic peptide comprises or consists of SEQ ID NO: 1; or
p) CDR3α comprises or consists of the amino acid sequence SEQ ID NO: 19, CDR3β comprises or consists of the amino acid sequence SEQ ID NO: 49, and wherein the DNAJB1-PRKACA antigenic peptide comprises or consists of SEQ ID NO: 5; or
q) CDR3α comprises or consists of the amino acid sequence SEQ ID NO: 19, CDR3β comprises or consists of the amino acid sequence SEQ ID NO: 62, and wherein the DNAJB1-PRKACA antigenic peptide comprises or consists of SEQ ID NO: 5; or
r) CDR3α comprises or consists of the amino acid sequence SEQ ID NO: 20, CDR3β comprises or consists of the amino acid sequence SEQ ID NO: 50, and wherein the DNAJB1-PRKACA antigenic peptide comprises or consists of SEQ ID NO: 5; or
s) CDR3α comprises or consists of the amino acid sequence SEQ ID NO: 21, CDR3β comprises or consists of the amino acid sequence SEQ ID NO: 51, and wherein the DNAJB1-PRKACA antigenic peptide comprises or consists of SEQ ID NO: 5; or
t) CDR3α comprises or consists of the amino acid sequence SEQ ID NO: 21, CDR3β comprises or consists of the amino acid sequence SEQ ID NO: 63, and wherein the DNAJB1-PRKACA antigenic peptide comprises or consists of SEQ ID NO: 5; or
u) CDR3α comprises or consists of the amino acid sequence SEQ ID NO: 22, CDR3β comprises or consists of the amino acid sequence SEQ ID NO: 52, and wherein the DNAJB1-PRKACA antigenic peptide comprises or consists of SEQ ID NO: 5; or
v) CDR3α comprises or consists of the amino acid sequence SEQ ID NO: 23, CDR3β comprises or consists of the amino acid sequence SEQ ID NO: 53, and wherein the DNAJB1-PRKACA antigenic peptide comprises or consists of SEQ ID NO: 5; or
w) CDR3α comprises or consists of the amino acid sequence SEQ ID NO: 24, CDR3β comprises or consists of the amino acid sequence SEQ ID NO: 54, and wherein the DNAJB1-PRKACA antigenic peptide comprises or consists of SEQ ID NO: 5; or
x) CDR3α comprises or consists of the amino acid sequence SEQ ID NO: 25, CDR3β comprises or consists of the amino acid sequence SEQ ID NO: 55, and wherein the DNAJB1-PRKACA antigenic peptide comprises or consists of SEQ ID NO: 5; or
y) CDR3α comprises or consists of the amino acid sequence SEQ ID NO: 34, CDR3β comprises or consists of the amino acid sequence SEQ ID NO: 55, and wherein the DNAJB1-PRKACA antigenic peptide comprises or consists of SEQ ID NO: 5; or
z) CDR3α comprises or consists of the amino acid sequence SEQ ID NO: 26, CDR3β comprises or consists of the amino acid sequence SEQ ID NO: 56, and wherein the DNAJB1-PRKACA antigenic peptide comprises or consists of SEQ ID NO: 5; or
α) CDR3α comprises or consists of the amino acid sequence SEQ ID NO: 27, CDR3β comprises or consists of the amino acid sequence SEQ ID NO: 57, and wherein the DNAJB1-PRKACA antigenic peptide comprises or consists of SEQ ID NO: 5; or
β) CDR3α comprises or consists of the amino acid sequence SEQ ID NO: 28, CDR3β comprises or consists of the amino acid sequence SEQ ID NO: 58, and wherein the DNAJB1-PRKACA antigenic peptide comprises or consists of SEQ ID NO: 5; or
γ) CDR3α comprises or consists of the amino acid sequence SEQ ID NO: 29, CDR3β comprises or consists of the amino acid sequence SEQ ID NO: 59, and wherein the DNAJB1-PRKACA antigenic peptide comprises or consists of SEQ ID NO: 5; or
δ) CDR3α comprises or consists of the amino acid sequence SEQ ID NO: 30, CDR3β comprises or consists of the amino acid sequence SEQ ID NO: 60, and wherein the DNAJB1-PRKACA antigenic peptide comprises or consists of SEQ ID NO: 5; or
ε) CDR3α comprises or consists of the amino acid sequence SEQ ID NO: 31, CDR3β comprises or consists of the amino acid sequence SEQ ID NO: 61, and wherein the DNAJB1-PRKACA antigenic peptide comprises or consists of SEQ ID NO: 5;
and wherein said CDR sequences may comprise one or more amino acid mutation(s) selected from amino acid insertion, deletion and/or substitution.

According to the invention an "antigen binding protein" herein refers to polypeptides or binding proteins that are able to bind to at least one DNAJB1-PRKACA antigenic peptide, in particular, a peptide as provided herein.

According to the invention, "CDR" stands for 'complementary-determining region'. A CDR is part of the variable chains in immunoglobulins (antibodies) and T-cell receptors, generated by B-cells and T-cells respectively, where these molecules bind to their specific antigen. There are three CDRs (CDR1, CDR2 and CDR3), arranged non-consecutively, on the amino acid sequence of a variable domain of an antigen receptor. In a T-cell receptor (TCR) the CDRs are located on two different protein chains. In humans, in 95% of T cells the TCR consists of an alpha (α) chain and a beta (β) chain (encoded by TRA and TRB, respectively), whereas in 5% of T cells the TCR consists of gamma and delta (γ/δ) chains (encoded by TRG and TRD, respectively). In respect of the antigen binding protein according to the invention CDR3α and CDR3β represent the protein chains which, in the natural TCR counterpart, are part of the complementary-determining regions 3 on the α and β chain.

The inventors were able to identify and sequence multiple TCRs from *in vitro* and *in vivo* induced DNAJB1-PRKACA-specific T cells, capable of specifically binding to the peptides according to the invention, in particular to peptides comprising the amino acid sequences of SEQ ID NOS: 1 and 5. The features recited under a) to ε) indicate amino acid sequence combinations of the variable CDR3α and CDR3β chains of an engineered antigen binding protein, such as a recombinant TCR, capable of specifically binding to an DNAJB1-PRKACA antigenic peptide comprising the amino acid sequences SEQ ID NO: 1 or 5.

The antigen binding proteins provided by the inventors are listed in the following table 3.

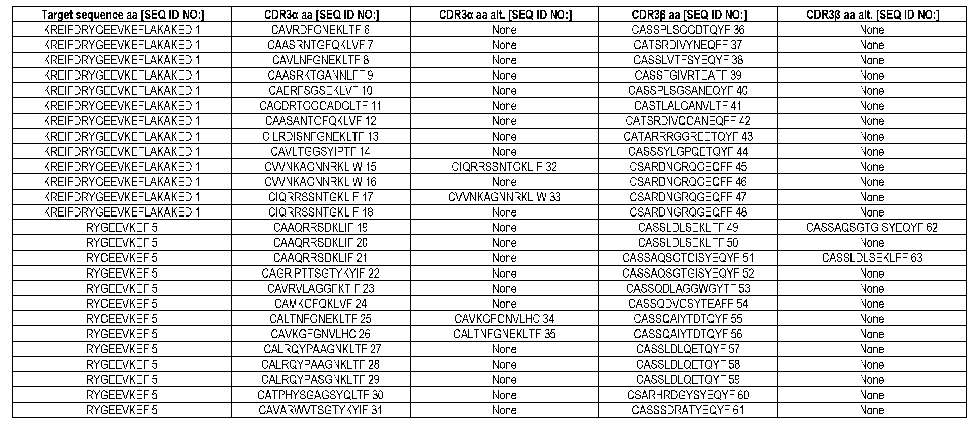

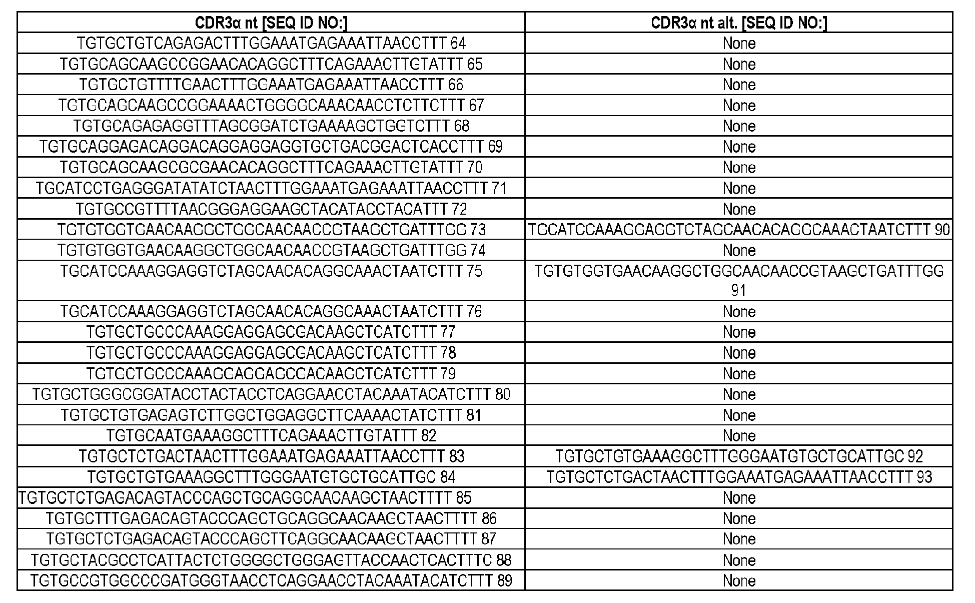

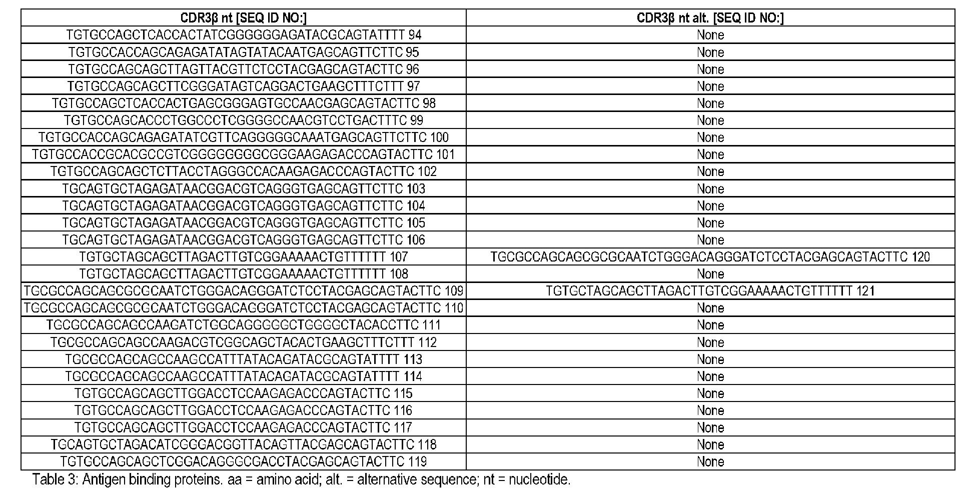

In the event of discrepancies between the sequences specified in Table 3 and those specified in the sequence listing, the information in the sequence listing takes precedence and applies.

It is to be understood that, in an embodiment of the antigen binding protein, said CDR sequences may comprise one or more amino acid mutation(s) selected from amino acid insertion, deletion and/or substitution. Preferably, such mutations do not, or, alternatively, not significantly affect the specific binding to the DNAJB1-PRKACA antigenic peptide. Further preferably, such mutations may be of a conservative nature, for example, where one amino acid is replaced by an amino acid of similar structure and characteristics, such as where a hydrophobic amino acid is replaced by another hydrophobic amino acid. Reference is made to the above disclosure regarding the modification and substitution of the peptides according to the invention which applies *mutatis mutandis* to the antigen binding protein according to the invention.

One or more in this context means 1, 2, 3, 4, or 5 etc. amino acid mutation(s).

The features, characteristics, advantages and embodiments disclosed for the peptide according to the invention apply to the antigen binding protein correspondingly.

In an embodiment of the invention the antigen binding protein is a single chain TCR (scTCR) or a single-chain bispecific antibody, and/or wherein the first polypeptide further comprises an α chain constant domain or a γ chain constant domain and the second polypeptide further comprises a β chain constant domain or a δ chain constant domain, and/or wherein said first variable domain is part of a TCR α chain or a TCR y chain and/or wherein said second variable domain is part of a TCR β chain or a TCR δ chain, and/or wherein the first variable domain is a TCR α variable domain, and the second variable domain is a TCR β variable domain, and/or wherein said antigen binding protein comprises at least one amino acid mutation conferring increased stability, surface expression and/or pairing.

"Single chain TCR (scTCR)" as used herein denotes a protein wherein the variable domains of the TCR, such as the Vα and Vβ or Vδ and Vγ are located on one polypeptide. Typically, the variable domains are separated by a linker, wherein said linker typically comprises 5 to 20 amino acids, such as 5 to 15 amino acids.

A "bispecific antibody" refers to a format which includes the non-limiting example of diabodies, Cross-Over-Dual-Variable-Domain (CODV) and/or in the Dual variable domain (DVD) proteins. An overview of these different bispecific antibodies and ways of making them is disclosed in, for example, Brinkmann U. and Kontermann E.E. MAbs. 2017 Feb-Mar; 9(2): 182-212. Particularly, the DVD format is, for example, disclosed in the following scientific articles (Wu C et al. Nat Biotechnol 2007; 25:1290-7; PMID:17934452; Wu C. et al. MAbs 2009; 1:339-47; Lacy SE et al. MAbs 2015; 7:605-19; PMID:25764208; Craig RB et al. PLoS One 2012; 7:e46778; 25 PMID:23056448; Piccione EC et al. MAbs 2015). The CODV is for example disclosed in Onuoha SC et al. Arthritis Rheumatol. 2015 Oct; 67(10):2661-72 or for example in WO2012/135345, WO2016/116626. Bispecific diabodies are for example described in Holliger P et al. Protein Eng 1996; 9:299-305; PMID:8736497; Atwell JL et al. Mol Immunol 1996; 33:1301-12; PMID:9171890; Kontermann RE, Nat Biotechnol 1997; 15:629-31; 30 PMID:9219263; Kontermann RE et al. Immunotechnology 1997; 3:137-44; PMID:9237098; Cochlovius B et al. Cancer Res 2000; 60:4336-41; PMID:10969772; and DeNardo DG et al. Cancer Biother Radiopharm 2001; 16:525-35; PMID:11789029.

The constant domain sequences of the antigen binding protein may be modified, for example, by the introduction of heterologous sequences, suitably mouse sequences, which may increase expression, pairing and stability. Mutations as known from the state of the art (e.g. WO 2018/104407, PCT/EP2018/069151, WO 2011/044186, WO 2014/018863, EP2432802B1 and WO2020157211A) may be introduced, such as replacement of unfavorable amino acids in the variable domains and/or the introduction of a disulfide bridge between the constant domains and the removal of unpaired cysteine.

In an embodiment of the invention the antigen binding protein further comprises one or more of the following:
(i) one or more further antigen binding sites;
(ii) a transmembrane region in said first polypeptide chain and/or said second polypeptide chain, optionally comprising a cytoplasmic signaling region;
(iii) a diagnostic agent;
(iv) a therapeutic agent; or
(v) a PK modifying moiety.

A "transmembrane region" may be, for example, a TCR alpha or beta transmembrane domain.

A "cytoplasmic signaling region" is, for example, a TCR alpha or beta intracellular domain.

A "diagnostic agent" herein refers to a detectable molecule or substance, such as a fluorescent molecule, a radioactive molecule or any other labels known in the art that provide (either directly or indirectly) a signal.

A "therapeutic agent" herein refers to an agent that has a therapeutic effect. In one embodiment, such a therapeutic agent may be a cytotoxic or cytostatic agent or any other anti-cancer agent, and/or an agent which induces an immune response which enhances the body's ability to fight cancer.

A "PK modifying moiety" herein refers to a moiety that modifies the pharmacokinetics (PK) of an antigen binding protein as described herein. Accordingly, the moiety modifies in particular the *in vivo* half-life and distribution of an antigen binding protein disclosed herein. In one embodiment, the PK modifying moiety increases the half-life of the antigen binding protein. Examples of PK modifying moieties include, but are not limited to, PEG (Dozier et al., 10 (2015) Int J Mol Sci. Oct 28;16(10):25831-64 and Jevsevar et al., (2010) Biotechnol J.Jan;5(1):113-28), PASylation (Schlapschy et al., (2013) Protein Eng Des Sel. Aug;26(8):489-501), albumin (Dennis et al., (2002) J Biol Chem. Sep 20;277(38):35035-43), the Fc-part of an antibody and/or unstructured polypeptides (Schellenberger et al., (2009) Nat Biotechnol. Dec; 27(12):1186-90).

In an embodiment of the invention the antigen binding protein is a therapeutic antigen binding protein and/or a diagnostic antigen binding protein.

Another subject-matter of the invention is an isolated nucleic acid molecule comprising a nucleotide sequence encoding the peptide or a variant thereof according to the invention, the antibody or fragment thereof according to the invention, the T-cell receptor according to the invention, or antigen binding protein according to the invention.

The nucleic acid coding for a particular peptide, oligopeptide, or polypeptide may be naturally occurring or they may be synthetically constructed. The nucleic acid (for example a polynucleotide) may be, for example, DNA, cDNA, PNA, RNA or combinations thereof, either single- and/or double- stranded, or native or stabilized forms of polynucleotides, such as, for example, polynucleotides with a phosphorothioate backbone and it may or may not contain introns so long as it codes for the peptide. Of course, only peptides that contain naturally occurring amino acid residues joined by naturally occurring peptide bonds are encodable by a polynucleotide. A still further aspect of the invention provides an expression vector capable of expressing the peptide according to the invention.

As used herein the term nucleic acid molecule or nucleotide sequence "encoding (or coding for)" a peptide etc. refers to a nucleotide sequence coding for the peptide including artificial (man-made) start and stop codons compatible for the biological system the sequence is to be expressed by, for example, a dendritic cell or another cell system useful for the production of TCRs. It is to be understood that the coding sequence is under the control of a region of nucleic acid, e.g. DNA, involved in binding of RNA polymerase to initiate transcription such as a "promoter". Therefore, the isolated nucleic acid molecule according to the invention may comprise regulatory elements such as a promoter, enhancer etc.

The term "isolated" has the meaning as defined further above.

In an embodiment of the invention the isolated nucleic acid molecule comprises a nucleotide sequence selected from the group consisting of SEQ ID NO: 64 to 119.

A yet other subject-matter of the invention relates to a cloning or expression vector comprising the isolated nucleic acid molecule according to the invention.

A still further subject-matter of the invention relates to a cloning or expression vector encoding an antigen binding protein which specifically binds at least one DNAJB1-PRKACA antigenic peptide in complex with a major histocompatibility complex (MHC) molecule, wherein said vector comprises, under the control of a promoter, a first nucleotide sequence which encodes a first variable domain that comprises at least one complementary determining region (CDR), CDR3α, and a second nucleotide sequence which encodes a second variable domain that comprises at least one CDR, CDR3β, wherein
a) the first nucleotide sequence comprises or consists of SEQ ID NO: 64, the second nucleotide sequence comprises or consists of SEQ ID NO: 94, and wherein the DNAJB1-PRKACA antigenic peptide comprises or consists of SEQ ID NO: 1; or
b) the first nucleotide sequence comprises or consists of SEQ ID NO: 65, the second nucleotide sequence comprises or consists of SEQ ID NO: 95, and wherein the DNAJB1-PRKACA antigenic peptide comprises or consists of SEQ ID NO: 1; or
c) the first nucleotide sequence comprises or consists of SEQ ID NO: 66, the second nucleotide sequence comprises or consists of SEQ ID NO: 96, and wherein the DNAJB1-PRKACA antigenic peptide comprises or consists of SEQ ID NO: 1; or
d) the first nucleotide sequence comprises or consists of SEQ ID NO: 67, the second nucleotide sequence comprises or consists of SEQ ID NO: 97, and wherein the DNAJB1-PRKACA antigenic peptide comprises or consists of SEQ ID NO: 1; or
e) the first nucleotide sequence comprises or consists of SEQ ID NO: 68, the second nucleotide sequence comprises or consists of SEQ ID NO: 98, and wherein the DNAJB1-PRKACA antigenic peptide comprises or consists of SEQ ID NO: 1; or
f) the first nucleotide sequence comprises or consists of SEQ ID NO: 69, the second nucleotide sequence comprises or consists of SEQ ID NO: 99, and wherein the DNAJB1-PRKACA antigenic peptide comprises or consists of SEQ ID NO: 1; or
g) the first nucleotide sequence comprises or consists of SEQ ID NO: 70, the second nucleotide sequence comprises or consists of SEQ ID NO: 100, and wherein the DNAJB1-PRKACA antigenic peptide comprises or consists of SEQ ID NO: 1; or
h) the first nucleotide sequence comprises or consists of SEQ ID NO: 71, the second nucleotide sequence comprises or consists of SEQ ID NO: 101, and wherein the DNAJB1-PRKACA antigenic peptide comprises or consists of SEQ ID NO: 1; or
i) the first nucleotide sequence comprises or consists of SEQ ID NO: 72, the second nucleotide sequence comprises or consists of SEQ ID NO: 102, and wherein the DNAJB1-PRKACA antigenic peptide comprises or consists of SEQ ID NO: 1; or
j) the first nucleotide sequence comprises or consists of SEQ ID NO: 73, the second nucleotide sequence comprises or consists of SEQ ID NO: 103, and wherein the DNAJB1-PRKACA antigenic peptide comprises or consists of SEQ ID NO: 1; or
k) the first nucleotide sequence comprises or consists of SEQ ID NO: 90, the second nucleotide sequence comprises or consists of SEQ ID NO: 103, and wherein the DNAJB1-PRKACA antigenic peptide comprises or consists of SEQ ID NO: 1; or
l) the first nucleotide sequence comprises or consists of SEQ ID NO: 74, the second nucleotide sequence comprises or consists of SEQ ID NO: 104, and wherein the DNAJB1-PRKACA antigenic peptide comprises or consists of SEQ ID NO: 1; or
m) the first nucleotide sequence comprises or consists of SEQ ID NO: 75, the second nucleotide sequence comprises or consists of SEQ ID NO: 105, and wherein the DNAJB1-PRKACA antigenic peptide comprises or consists of SEQ ID NO: 1; or
n) the first nucleotide sequence comprises or consists of SEQ ID NO: 91, the second nucleotide sequence comprises or consists of SEQ ID NO: 105, and wherein the DNAJB1-PRKACA antigenic peptide comprises or consists of SEQ ID NO: 1; or
o) the first nucleotide sequence comprises or consists of SEQ ID NO: 76, the second nucleotide sequence comprises or consists of SEQ ID NO: 106, and wherein the DNAJB1-PRKACA antigenic peptide comprises or consists of SEQ ID NO: 1; or
p) the first nucleotide sequence comprises or consists of SEQ ID NO: 77, the second nucleotide sequence comprises or consists of SEQ ID NO: 107, and wherein the DNAJB1-PRKACA antigenic peptide comprises or consists of SEQ ID NO: 5; or
q) the first nucleotide sequence comprises or consists of SEQ ID NO: 77, the second nucleotide sequence comprises or consists of SEQ ID NO: 120, and wherein the DNAJB1-PRKACA antigenic peptide comprises or consists of SEQ ID NO: 5; or
r) the first nucleotide sequence comprises or consists of SEQ ID NO: 78, the second nucleotide sequence comprises or consists of SEQ ID NO: 108, and wherein the DNAJB1-PRKACA antigenic peptide comprises or consists of SEQ ID NO: 5; or
s) the first nucleotide sequence comprises or consists of SEQ ID NO: 79, the second nucleotide sequence comprises or consists of SEQ ID NO: 109, and wherein the DNAJB1-PRKACA antigenic peptide comprises or consists of SEQ ID NO: 5; or
t) the first nucleotide sequence comprises or consists of SEQ ID NO: 79, the second nucleotide sequence comprises or consists of SEQ ID NO: 121, and wherein the DNAJB1-PRKACA antigenic peptide comprises or consists of SEQ ID NO: 5; or
u) the first nucleotide sequence comprises or consists of SEQ ID NO: 80, the second nucleotide sequence comprises or consists of SEQ ID NO: 110, and wherein the DNAJB1-PRKACA antigenic peptide comprises or consists of SEQ ID NO: 5; or
v) the first nucleotide sequence comprises or consists of SEQ ID NO: 81, the second nucleotide sequence comprises or consists of SEQ ID NO: 111, and wherein the DNAJB1-PRKACA antigenic peptide comprises or consists of SEQ ID NO: 5; or
w) the first nucleotide sequence comprises or consists of SEQ ID NO: 82, the second nucleotide sequence comprises or consists of SEQ ID NO: 112, and wherein the DNAJB1-PRKACA antigenic peptide comprises or consists of SEQ ID NO: 5; or
x) the first nucleotide sequence comprises or consists of SEQ ID NO: 83, the second nucleotide sequence comprises or consists of SEQ ID NO: 113, and wherein the DNAJB1-PRKACA antigenic peptide comprises or consists of SEQ ID NO: 5; or
y) the first nucleotide sequence comprises or consists of SEQ ID NO: 84, the second nucleotide sequence comprises or consists of SEQ ID NO: 114, and wherein the DNAJB1-PRKACA antigenic peptide comprises or consists of SEQ ID NO: 5; or
z) the first nucleotide sequence comprises or consists of SEQ ID NO: 93, the second nucleotide sequence comprises or consists of SEQ ID NO: 114, and wherein the DNAJB1-PRKACA antigenic peptide comprises or consists of SEQ ID NO: 5; or
α) the first nucleotide sequence comprises or consists of SEQ ID NO: 85, the second nucleotide sequence comprises or consists of SEQ ID NO: 115, and wherein the DNAJB1-PRKACA antigenic peptide comprises or consists of SEQ ID NO: 5; or
β) the first nucleotide sequence comprises or consists of SEQ ID NO: 86, the second nucleotide sequence comprises or consists of SEQ ID NO: 116, and wherein the DNAJB1-PRKACA antigenic peptide comprises or consists of SEQ ID NO: 5; or
γ) the first nucleotide sequence comprises or consists of SEQ ID NO: 87, the second nucleotide sequence comprises or consists of SEQ ID NO: 117, and wherein the DNAJB1-PRKACA antigenic peptide comprises or consists of SEQ ID NO: 5; or
δ) the first nucleotide sequence comprises or consists of SEQ ID NO: 88, the second nucleotide sequence comprises or consists of SEQ ID NO: 118, and wherein the DNAJB1-PRKACA antigenic peptide comprises or consists of SEQ ID NO: 5; or
ε) the first nucleotide sequence comprises or consists of SEQ ID NO: 89, the second nucleotide sequence comprises or consists of SEQ ID NO: 119, and wherein the DNAJB1-PRKACA antigenic peptide comprises or consists of SEQ ID NO: 5;
and wherein said first and/or second nucleotide sequences may comprise one or more nucleic acid mutation(s) selected from nucleotide insertion, deletion and/or substitution.

The cloning or expression vector according to the invention serve as a basic tool to produce the specific antigen binding proteins according to the invention capable of specifically binding to the peptides according to the invention, in particular to peptides comprising the amino acid sequences of SEQ ID NOS: 1 and 5. The remaining features recited under a) to ε) indicate nucleotide sequence combinations encoding the variable CDR3α and CDR3β chains of an to-be-engineered antigen binding protein, such as a recombinant TCR, capable of specifically binding to an DNAJB1-PRKACA antigenic peptide comprising the amino acid sequences SEQ ID NO: 1 or 5.

"One or more" in this context means 1, 2, 3, 4, 5, 6, 7, 8, 9, 10 etc. nucleic acid mutation(s).

The cloning or expression vectors provided by the inventors are listed in the above table 3.

The features, characteristics, advantages and embodiments disclosed for the peptide according to the invention apply to the nucleic acid molecule and the cloning or expression vectors correspondingly.

Another subject-matter of the invention relates to a recombinant host cell comprising the peptide according to the invention, the antibody or fragment thereof according to the invention, the T cell according to the invention , the antigen binding protein according to the invention, the isolated nucleic acid or the vector according to the invention, wherein said host cell preferably is selected from a mammalian or human cell, further preferably from an antigen presenting cell, such as a dendritic cell, a T cell or an NK cell.

The features, characteristics, advantages and embodiments disclosed for the peptide according to the invention apply to the host cell correspondingly.

A still further subject-matter of the invention relates to an *in vitro* method for producing activated T cells, the method comprising contacting *in vitro* T cells with antigen loaded human class I or II MHC molecules expressed on the surface of a suitable antigen-presenting cell or an artificial construct mimicking an antigen-presenting cell for a period of time sufficient to activate said T cells in an antigen specific manner, wherein said antigen is a peptide according to the invention.

The activated T cells that are directed against the peptides of the invention are useful in therapy. Thus, a further aspect of the invention provides activated T cells obtainable by the foregoing methods of the invention.

Activated T cells, which are produced by the above method, will selectively recognize a cell that aberrantly expresses a polypeptide that comprises an amino acid sequence of SEQ ID NO: 1 to SEQ ID NO: 5.

The features, characteristics, advantages and embodiments disclosed for the peptide according to the invention apply to the above method correspondingly.

Another subject-matter according to the invention relates to a pharmaceutical composition comprising at least one active ingredient selected from the group consisting of the peptide or a variant thereof according to the invention, the antibody or fragment thereof according to the invention, the T-cell receptor according the invention, the antigen binding protein according to any of the invention, the isolated nucleic acid molecule according to the invention, or the vector according to the invention, the recombinant host cell according to the invention, or the activated T lymphocyte according to the invention, or a conjugated or labelled active ingredient, and a pharmaceutically acceptable carrier, and optionally, pharmaceutically acceptable excipients and/or stabilizers.

A "pharmaceutical composition" is a composition suitable for administration to a human being in a medical setting. Preferably, a pharmaceutical composition is sterile and produced according to GMP guidelines.

The pharmaceutical compositions comprise the peptides either in the free form or in the form of a pharmaceutically acceptable salt (see also above). As used herein, "a pharmaceutically acceptable salt" refers to a derivative of the disclosed peptides wherein the peptide is modified by making acid or base salts of the agent. For example, acid salts are prepared from the free base (typically wherein the neutral form of the drug has a neutral -NH₂ group) involving reaction with a suitable acid. Suitable acids for preparing acid salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methane sulfonic acid, ethane sulfonic acid, p-toluenesulfonic acid, salicylic acid, and the like, as well as inorganic acids, e.g., hydrochloric acid, hydrobromic acid, sulfuric acid, nitric acid phosphoric acid and the like. Conversely, preparation of basic salts of acid moieties which may be present on a peptide are prepared using a pharmaceutically acceptable base such as sodium hydroxide, potassium hydroxide, ammonium hydroxide, calcium hydroxide, trimethylamine or the like.

Preferably, the pharmaceutical composition of the present invention is an immunotherapeutic such as a vaccine. It may be administered directly into the patient, into the affected organ or systemically i.d., i.m., s.c., i.p. and i.v., or applied *ex vivo* to cells derived from the patient or a human cell line which are subsequently administered to the patient, or used *in vitro* to select a subpopulation of immune cells derived from the patient, which are then re-administered to the patient. If the nucleic acid is administered to cells *in vitro*, it may be useful for the cells to be transfected so as to co-express immune-stimulating cytokines, such as interleukin-2.

The peptide may be substantially pure. The peptide may also be combined with an immune-stimulating adjuvant, such as the TLR1/2 ligand XS15. Preliminary work has shown that XS15 can induce a strong CD8⁺ and Th1CD4⁺ T-cell response *in vivo* after subcutaneous injection in healthy donors and individual tumor patients for viral, mutated and unmutated peptides in a water-oil emulsion. The peptide may also be used in combination with immune-stimulatory cytokines, or be administered with a suitable delivery system, for example liposomes. The peptide may also be conjugated to a suitable carrier such as keyhole limpet haemocyanin (KLH) or mannan (see WO 95/18145 and (Longenecker et al., Ann. N.Y. Acad. Sci. 690:276-291 (1993)). The peptide may also be tagged, may be a fusion protein, or may be a hybrid molecule. The peptides whose sequence is given in the present invention are expected to stimulate CD4 or CD8 T cells. However, stimulation of CD8 T cells is more efficient in the presence of help provided by CD4 T-helper cells. Thus, for MHC Class I epitopes that stimulate CD8 T cells the fusion partner or sections of a hybrid molecule suitably provide epitopes which stimulate CD4-positive T cells. CD4- and CD8-stimulating epitopes are well known in the art and include those identified in the present invention. In one aspect, the vaccine comprises at least one peptide having the amino acid sequence set forth SEQ ID NO: 1 to SEQ ID NO: 5, and at least one additional peptide, preferably two to 50, more preferably two to 25, even more preferably two to 20 and most preferably two, three, four, five, six, seven, eight, nine, ten, eleven, twelve, thirteen, fourteen, fifteen, sixteen, seventeen or eighteen peptides. The peptide(s) may be derived from one or more specific TAAs and may bind to MHC class I molecules.

In an embodiment of the pharmaceutical composition according to the invention it comprises at least 1, preferably at least 2, further preferably at least 3, further preferably at least 4, further preferably 5 peptides according to the invention. For a "universal" pharmaceutical composition or vaccine the inclusion of a peptide comprising the amino acid sequences of SEQ ID NO: 1 and/or 2 or variants thereof is preferred as such peptides can be administered to individuals independent of their MHC or HLA allotypes. Peptides comprising the amino acid sequences of SEQ ID NO: 3 and/or 4 and/or 5 or variants thereof are preferably included into a pharmaceutical composition to be administered to individuals with an MHC or HLA allotype of A*68:02 (SEQ ID NO: 3), C*04:01;C*05:01 (SEQ ID NO: 4) or A*24:02 (SEQ ID NO: 5).

The pharmaceutical composition according to the invention may comprise, as the only active ingredient, the peptide according to the invention, the antibody or fragment thereof according to the invention, the T-cell receptor according to the invention, the antigen binding protein according to the invention, the nucleic acid or the expression vector according to the invention, the recombinant host cell according to the invention, or the activated T lymphocyte according to the invention. However, in an alternative aspect, it may comprise an additional active ingredient such as a small molecule, e.g. ibrutinib and/or ideasilib and/or venetoclax.

Another subject-matter of the invention relates to a peptide or a variant thereof according to the invention, the antibody or fragment thereof according to the invention, the T-cell receptor according the invention, the antigen binding protein according to the invention, the isolated nucleic acid molecule according to the invention, or the vector according to the invention, the recombinant host cell according to the invention, or the activated T cell according to the invention for use in medicine, preferably said use is in diagnosis and/or prevention and/or treatment of cancer, or for use in the manufacture of a medicament against cancer, further preferably said cancer is a cancer with a fusion of DnaJ heat shock protein family member B1 (*DNAJB1*) and protein kinase cAMP-activated catalytic subunit alpha (*PRKACA*) (DNAJB1-PRKACA), highly preferably fibrolamellar hepatocellular carcinoma (FL-HCC), and other cancers that show an overexpression of a protein from which a peptide comprising SEQ ID NO: 1 to SEQ ID NO: 5 is derived from.

The features, characteristics, advantages and embodiments disclosed for the peptide according to the invention apply to the above-referenced use correspondingly.

A still further subject-matter of the invention relates to a kit comprising:
(a) a container comprising a pharmaceutical composition containing the peptide or a variant thereof according to the invention, the antibody or fragment thereof according to the invention, the T-cell receptor according to the invention, the antigen binding protein according to the invention, the isolated nucleic acid molecule according to the invention, or the vector according to the invention, the recombinant host cell according to the invention, or the activated T cell according to the invention in solution or in lyophilized form,
(b) optionally, a second container containing a diluent or reconstituting solution for the lyophilized formulation;
(c) optionally, at least one more peptide selected from the group consisting of SEQ ID NO: 1 to SEQ ID NO: 5, and
(d) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The pharmaceutical composition is preferably lyophilized.

The kit of the present invention preferably comprises a lyophilized formulation of the present invention in a suitable container and instructions for its reconstitution and/or use. Suitable containers include, for example, bottles, vials (e.g. dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

The features, characteristics, advantages and embodiments disclosed for the peptide according to the invention apply to the kit correspondingly

Another subject-matter of the invention relates to a method for producing a personalized anti-cancer vaccine, said method comprising:
a) identifying tumor-associated peptides (TUMAPs) presented by a tumor sample from an individual patient;
b) comparing the peptides as identified in step a) with a warehouse of peptides which have been pre-screened for immunogenicity and/or over-presentation in tumors as compared to normal tissues;
c) selecting at least one peptide from the warehouse that matched a TUMAP identified in said patient; and
d) formulating the personalized vaccine based on step c),
wherein said warehouse comprises at least one of the peptide or variant thereof according to the invention.

A "personalized pharmaceutical composition" shall mean specifically tailored therapies for one individual patient that will only be used for therapy in such individual patient, including actively personalized cancer vaccines and adoptive cellular therapies using autologous patient tissue.

As used herein, the term "warehouse" shall refer to the peptides according to the invention that have been pre-screened for immunogenicity and/or over-presentation in a particular tumor type, in particular a cancer with a fusion of DnaJ heat shock protein family member B1 (DNAJB1) and protein kinase cAMP-activated catalytic subunit alpha (PRKACA) (DNAJB1-PRKACA). The warehouse (e.g. in the form of a database) is composed of tumor-associated peptides which were highly overexpressed in cancer cells of patients with various HLA-A HLA-B and HLA-C alleles. It contains MHC class I and MHC class II peptides. In particular, it contains MHC class I A*24, B*07, and A*02 marker peptides. These peptides allow comparison of the magnitude of T-cell immunity induced by TUMAPS in a quantitative manner and hence allow important conclusion to be drawn on the capacity of the vaccine to elicit anti-tumor responses.

In one exemplary embodiment, the peptides included in the vaccine are identified by: (a) identifying tumor-associated peptides (TUMAPs) presented by a tumor sample from the individual patient by the method as described above; (b) comparing the peptides identified in a) with the warehouse of peptides that have been prescreened for immunogenicity and overpresentation in tumors as compared to corresponding normal tissue; (c) selecting at least one peptide from the warehouse that correlates with a tumor-associated peptide identified in the patient; and (d) optionally, selecting at least one peptide identified de novo in (a) confirming its immunogenicity.

Once the peptides for a personalized peptide based pharmaceutical composition, e.g. a vaccine, are selected, the composition is produced. The composition or vaccine preferably is a liquid formulation consisting of the individual peptides dissolved in between 20-40% DMSO, preferably about 30-35% DMSO, such as about 33% DMSO.

The features, characteristics, advantages and embodiments disclosed for the peptide according to the invention apply to the afore-referenced method correspondingly.

It is to be understood that the before-mentioned features and those to be mentioned in the following cannot only be used in the combination indicated in the respective case, but also in other combinations or in an isolated manner without departing from the scope of the invention.

### EMBODIMENTS

The invention is now further explained by means of embodiments resulting in additional features, characteristics and advantages of the invention. The embodiments are of pure illustrative nature and do not limit the scope or range of the invention. The features mentioned in the specific embodiments are features of the invention and may be seen as general features which are not applicable in the specific embodiment but also in an isolated manner in the context of any embodiment of the invention.

The invention is now further described and explained in further detail by referring to the following non-limiting examples and Figures:

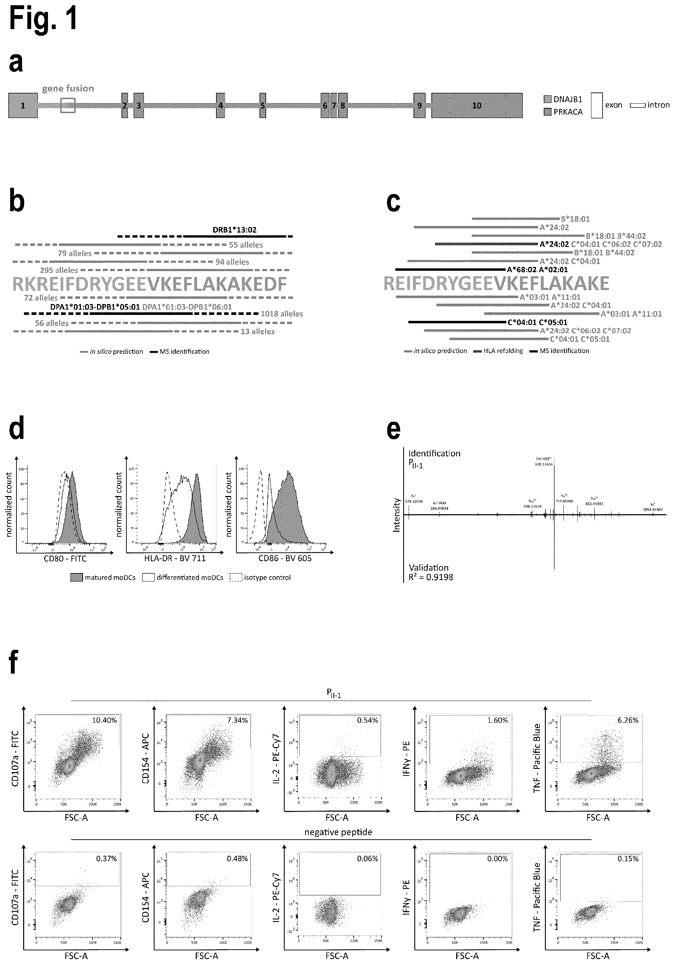

- Fig. 1:: Prediction of *DNAJB1-PRKACA* fusion gene-derived HLA class I and II ligands and characterization of a DNAJB1-PRKACA-*derived* CD4⁺ T-cell epitope. a, Schematic overview of the *DNAJB1-PRKACA* fusion transcript with exon 1 from *DNAJB1* and exon 2-10 from *PRKACA.* b, DNAJB1-PRKACA protein fusion region HLA class II ligand prediction, indicating in grey and black *in silico* predicted and MS identified ligands, respectively. Continuous lines illustrate the 9 amino acid binding cores, dashed lines the up to 15mer HLA ligand extensions. Allele numbers depict the number of HLA alleles predicted to bind to the respective core sequence. c, DNAJB1-PRKACA protein fusion region HLA class I ligand prediction, indicating in grey *in silico* predicted, in black bordered HLA refolded, and in black mass spectrometry-identified ligands. d, Flow cytometry-based characterization of mature monocyte-derived dendritic cells (moDCs) in comparison to differentiated moDCs and isotype control with indicated surface markers. e, Comparison of fragment spectra (m/z on x-axis) of the experimentally eluted peptide KREIFDRYGEEVKEFLAKAKED (P_{II-1}) (SEQ ID NO: 1) extracted from peptide-loaded mature moDCs (identification) with the directly measured synthetic peptide (validation, mirrored on the x-axis), both isotopically labelled on amino acid position 4, 12, and 19. f, Representative example of flow cytometry-based functional characterization with indicated cytokines and surface markers of P_{II-1}-specific CD4⁺ T cells derived from a healthy volunteer after *de novo* priming with P_{II-1}-loaded mature moDCs (upper panel). As negative control, P_{II-1}-primed CD4⁺ T cells stimulated with negative peptide (lower panel).

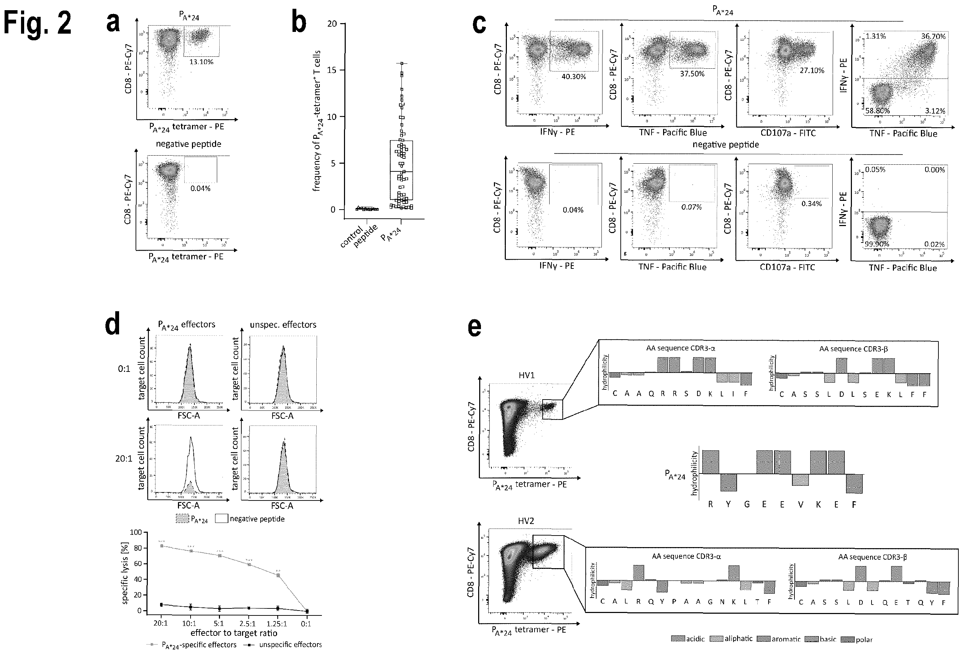

- Fig. 2:: Characterization of a DNAJB1-PRKACA-*derived* CD8⁺ T-cell epitope and single-cell TCR sequencing. a, Representative example of flow cytometry-based characterization of P_{A*24}-specific CD8⁺ T cells of a healthy volunteer (HV) after *in vitro* artificial antigen presenting cell (aAPC)-priming with HLA-A*24- P_{A*24}-monomer. b, Frequencies of P_{A*24}-specific CD8⁺ T cells compared to CD8⁺ T cells primed with a HLA-matched negative peptide in HVs (n = 5). c, Representative example of IFN⊐ and TNF production as well as CD107a expression of P_{A*24}-specific CD8⁺ T cells stimulated with P_{A*24} (upper panel) or a HLA-matched negative peptide (lower panel). d, Specific cell lysis by P_{A*24}-specific CD8⁺ T cells of P_{A*24}-loaded autologous CD8⁻ target cells (grey fill, dashed line (upper panel); red line (lower panel)) at various effector-to-target cell ratios compared to negative peptide-loaded autologous CD8⁻ target cells (white fill, solid line (upper panel)). P_{A*24}-unspecific CD8⁺ T cells showed no lysis of the target cells (black line (lower panel). Results are shown as mean ± SD for three independent technical replicates. Two-tailed paired student's t-test (***P < 0.01; ***P < 0.001*). e, Flow cytometry-based bulk sort of P_{A*24}-specific CD8⁺ T cells of two HVs (HV1, HV2) after aAPC-based priming with HLA-A*24-P_{A*24}-monomer (left panel) for single-cell TCR sequencing. The right panel depicts physiochemical properties and amino acid sequences of the CDR3-α/-β region of the most frequent T-cell receptor (TCR)-clone from each donor in comparison to their target peptide P_{A*24}. On the y-axes the hydrophilicity according to the Hopp-Woods scale is indicated and amino acids (AA) are grouped by their physiochemical properties with color code.

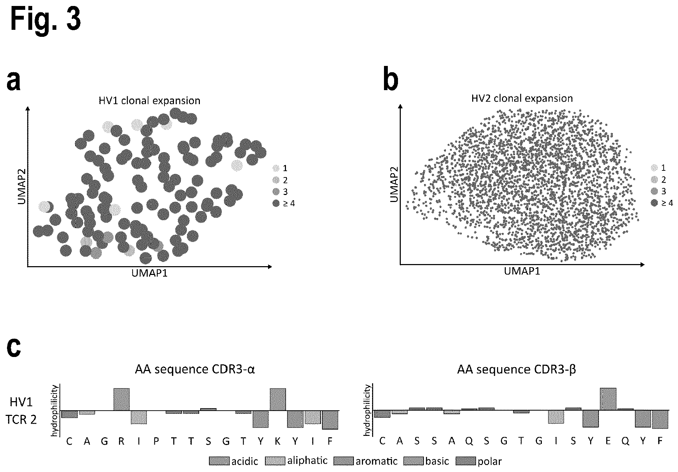

- Fig. 3:: Single-cell TCR sequencing of P_{A*24}-specific CD8⁺ T cells. (a-c) Single-cell RNA and TCR sequencing was performed for tetramer-sorted P_{A*24}-specific CD8⁺ T cells of two healthy volunteers (HV) after artificial antigen presenting cell (aAPC)-based priming with HLA-A*24-P_{A*24}-monomer. (a, b) Uniform Manifold Approximation and Projection (UMAP) plots showing T-cell receptor (TCR) clonality of P_{A*24}-specific CD8⁺ T cells of a, HV1 and b, HV2. The color code indicates the number of cells belonging to an expanded clonotype c, Physiochemical properties and amino acid (AA) sequences of the CDR3-α/-β region of the second most frequent TCR-clone of HV1. Hydrophilicity according to the Hopp-Woods scale is indicated on the y-axes. AAs are grouped by their physiochemical properties with color code.

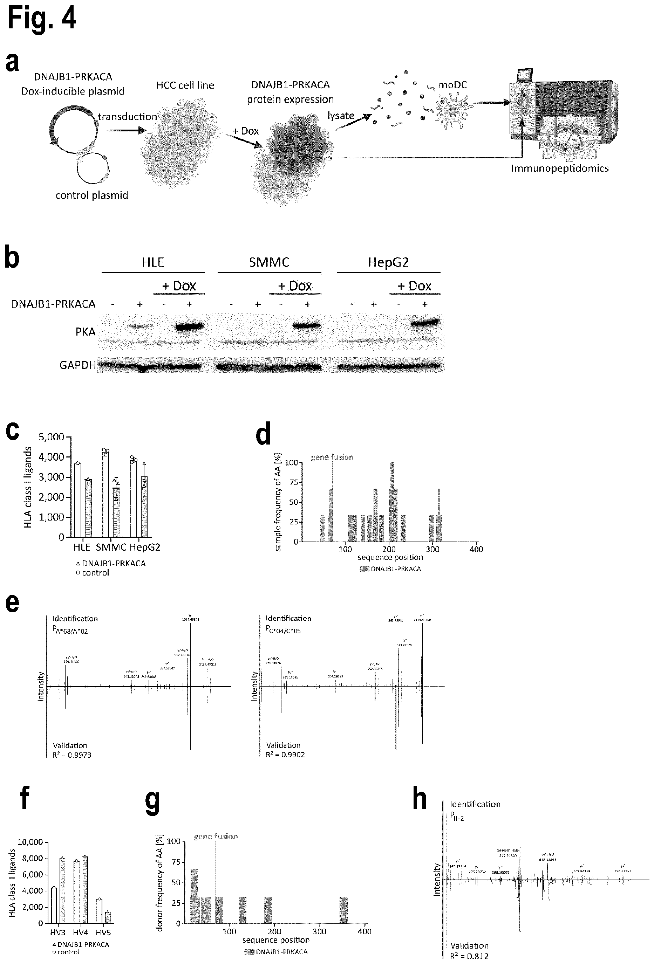

- Fig. 4:: Mass-spectrometry based identification of naturally-presented DNAJB1-PRKACA-derived HLA class I and class II ligands. a, Schematic overview of the experimental setup for Doxycycline (Dox)-inducible *DNAJB1-PRKACA* fusion gene expression. Hepatocellular carcinoma (HCC) cell lines transduced with the *DNAJB1-PRKACA* Dox-inducible plasmid or a control plasmid were treated with Dox, followed by mass-spectrometry (MS)-based immunopeptidome analysis of the HCC cell line itself or of mature monocyte-derived dendritic cells (moDC) of a healthy volunteer (HV) incubated with HCC cell line lysate. b, Dox induced DNAJB1-PRKACA fusion protein expression by immunoblotting of HCC cell lines (HLE, SMMC-7721, HepG2) carrying the *DNAJB1-PRKACA* Dox-inducible plasmid (+) or the control plasmid (-), with and without Dox treatment. GAPDH served as loading control. (c, e) MS-identified HLA class I and HLA class II peptides of c, HCC cell lines carrying the *DNAJB1-PRKACA* Dox-inducible or the control plasmid after Dox-treatment and e, of mature moDCs of HVs incubated with HCC cell line lysate with or without expression of DNAJB1-PRKACA protein, respectively. (d, f) Frequency of amino acids (AA) per sample distributed over the DNAJB1-PRKACA fusion protein sequence of d, HLA class I ligands of the HCC cell lines (n = 3) expressing the DNAJB1-PRKACA protein and f, HLA class II peptides of mature moDCs of HVs (n = 3) incubated with HCC cell line lysate with expression of DNAJB1-PRKACA protein. (g, h) Fragment spectra (m/z on x-axis) of the experimentally eluted peptides g, EIFDRYGEEV (P_{A*68/A*02}, left) (SEQ ID NO: 3) and IFDRYGEEV (P_{C*04/C*05}, right) (SEQ ID NO: 4) extracted from the *DNAJB1-PRKACA* expressing cell lines SMMC-7721 or HepG2, respectively (identification) compared to the respective synthetic peptide (validation, mirrored on the x-axis) and h, EVKEFLAKAKEDFLKK (P_{II-2}) (SEQ ID NO: 2) extracted from mature moDCs of a HV incubated with HCC cell line lysate expressing the DNAJB1-PRKACA protein (identification) compared to the respective synthetic peptide isotopically labelled on amino acid position two (validation, mirrored on the x-axis).

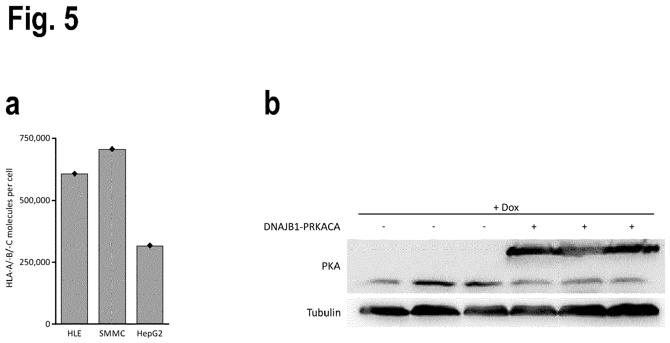

- Fig. 5:: HCC cell lines expressing the DNAJB1-PRKACA fusion protein. a, Flow cytometry-based quantification of HLA class I molecule surface expression for hepato-cellular carcinoma (HCC) cell lines HLE, SMMC-7721, and HepG2. b, Determination of Doxycycline (Dox)-induced DNAJB1-PRKACA fusion protein expression by immunoblotting of cell lysates used for monocyte-derived dendritic cells loading of the HCC cell line HLE carrying the *DNAJB1-PRKACA* Dox-inducible plasmid (+) or the control plasmid (-), after Dox treatment. Tubulin served as loading control.

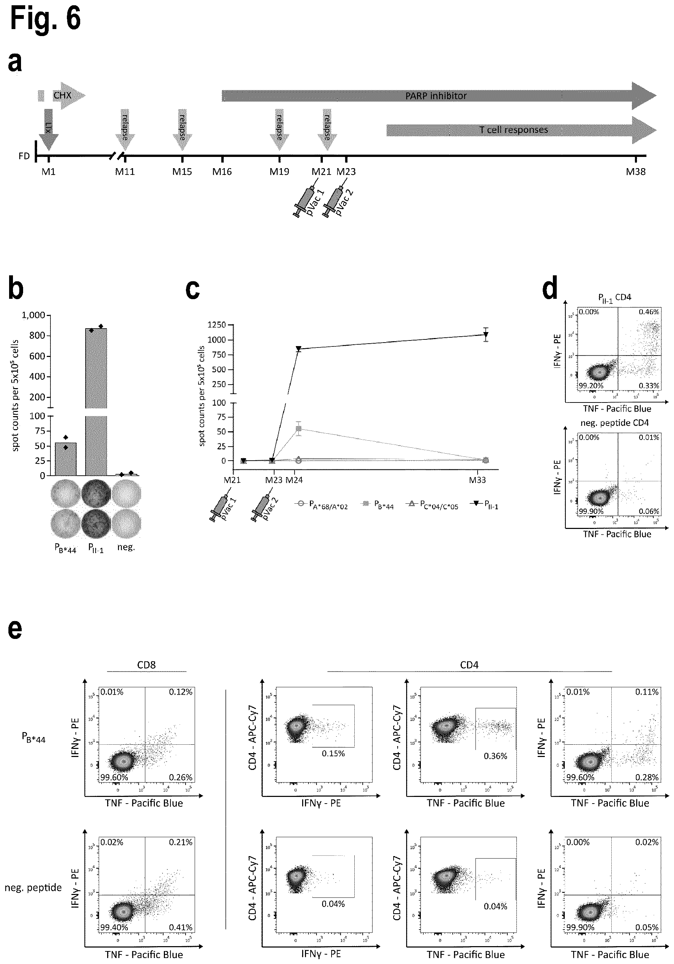

- Fig. 6:: FL-HCC patient vaccinated with a personalized DNAJB1-PRKACA-derived peptide vaccine. a, Schematic therapy course of a fibrolamellar hepatocellular carcinoma (FL-HCC) patient treated with a DNAJB1-PRKACA-derived peptide vaccine cocktail. After first diagnosis (FD) the patient was treated with four cycles of chemotherapy (CHX) analogous to the PHITT study (PHITT Group F), interrupted by early liver transplant (LTx) one month (M1) after FD, as the tumor was assessed not resectable. Everolimus was used for post transplantation immuno-suppression. The patient experienced four relapses after LTx at month 11, 15, 19, and 21 post FD. Tumor manifestations of the first, second and fourth relapse were surgically resected, for the third relapse radiotherapy was applied. Starting at month 16 post FD the patient was treated with Olaparib (poly ADP ribose polymerase (PARP) inhibitor). At month 21 and 23 the patient received two vaccinations of a personalized DNAJB1-PRKACA-derived peptide vaccine comprising P_{A*68/A*02}, P_{B*44}, P_{C*04/C*05}, and P_{II-1}. Induction of vaccine-peptide specific T-cell responses was observed six weeks after the second vaccination. b, Vaccine peptide-specific T-cell responses six weeks after the second vaccination assessed by IFNγ ELISPOT assay after *in vitro* stimulation with the vaccine cocktail peptides (P_{B*44}, P_{II-1}), compared to the negative peptide (neg.). c, Longitudinal analysis of vaccine-induced T-cell responses up to 10 months post vaccination using IFNγ ELISPOT assay after *in vitro* stimulation with the vaccine cocktail peptides (P_{A*68/A*02}, P_{B*44}, P_{C*04/C*05}, and P_{II-1)}. (d, e) Flow cytometry-based characterization for indicated cytokines of d, CD4⁺ T cells stimulated with the P_{II-1} peptide and e, CD4⁺ and CD8⁺ T cells stimulated with the P_{B*44} peptide 14 weeks after the second vaccination, in comparison to the respective negative (neg.) peptides.

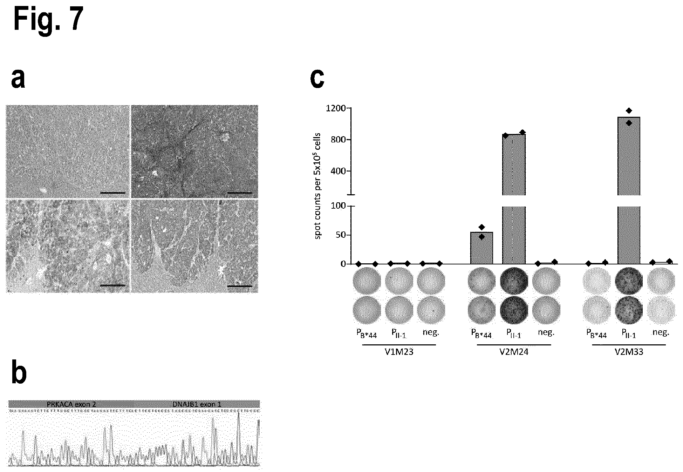

- Fig. 7:: Characterization of primary tumor cells and sequential immunomonitoring of a FL-HCC patient vaccinated with a personalized DNAJB1-PRKACA-derived peptide vaccine. a Representative micrographs of the FL-HCC patient at diagnosis. Upper left panel, HE staining; upper right panel, Masson's Trichrome staining; lower left panel, CK7 immunohistochemical staining; and lower right panel, Hepar1 immunohistochemical staining. Scale bar = 500 µm, magnification x 40. b, Sanger sequencing of the reverse transcription polymerase chain reaction (RTPCR) product for confirmation of the chimera transcript joining the end of exon 1 of DNAJB1 and the start of exon 2 of PRKACA. c, IFNγ ELISPOT assay of peptide-specific T cells from the FL-HCC patient after *in vitro* stimulation with the vaccine cocktail peptides P_{B*44}, and P_{II-1} six weeks after the first vaccination (V1M23), six weeks after the second vaccination (V2M24), and 45 weeks after the second vaccination (V2M33), compared to the negative peptide (neg.).

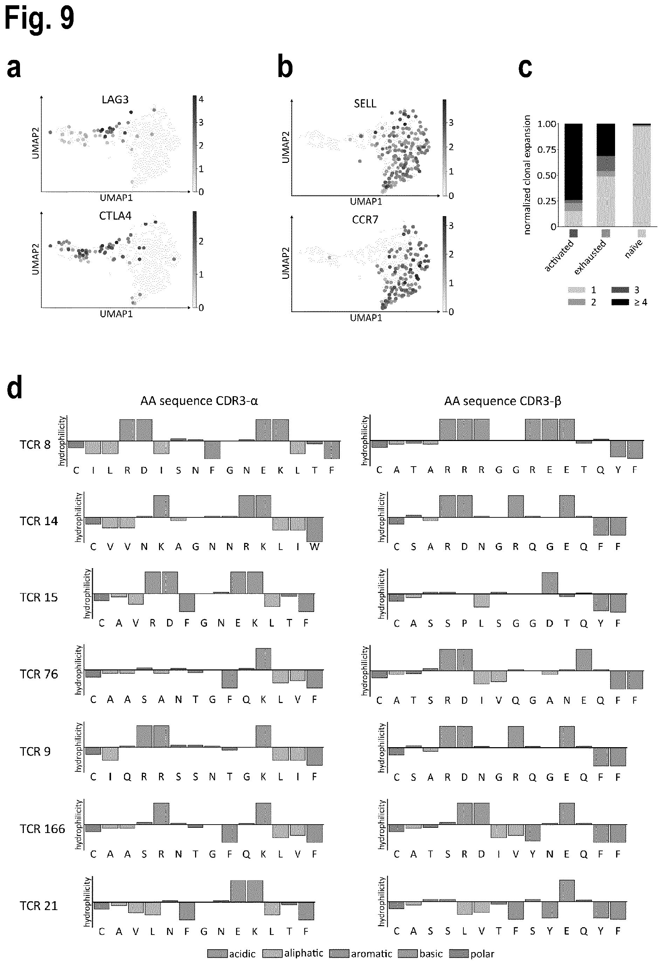

- Fig. 9:: Single-cell RNA sequencing of vaccine-induced P_{II-1} specific CD4⁺ T cells. (a-e), Single-cell RNA sequencing analysis of CD4⁺ T cells sorted from P_{II-1} stimulated PBMCs of the FL-HCC patient 31 weeks after the second vaccination with a personalized DNAJB1-PRKACA-derived peptide vaccine. a Uniform Manifold Approximation and Projection (UMAP) plots showing distinct T-cell clusters b, Heat map of cluster defining genes for activated, exhausted/late effector and naive resting T cells. c, Feature plots showing *IFNG*, *GZMB* (encoding granzyme B), *TNF*, and *CCL3* gene expression defining the activated T-cell cluster. d, UMAP plot showing T-cell receptor (TCR) clonality of sequenced CD4⁺ T cells. The color code indicates the number of cells belonging to an expanded clonotype. e Distribution of the ten largest TCR clonotypes across the cell type clusters identified in P_{II-1}-specific CD4⁺ T cells. f Physiochemical properties and amino acid sequences of the CDR3-α/-β region of the most frequent TCR-clones in comparison to their target peptide P_{II-1}. The y-axes indicate the hydrophilicity according to the Hopp-Woods scale. Amino acids (AA) are grouped by their physiochemical properties with color code.

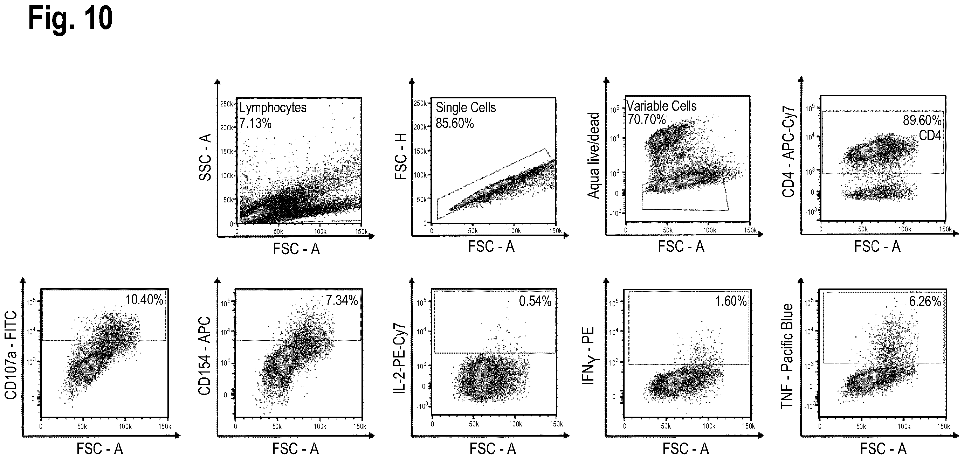

- Fig. 10:: Gating strategy for the ICS evaluation of moDC primed CD4⁺ T cells. Exemplary sample showing the gating strategy for evaluation of intracellular cytokine stainings (ICS) of monocyte-derived dendritic cells (moDC) primed CD4⁺ T cells. The first gate identifies the lymphocytes (FSC-A vs. SSC-A), which are further gated for single cells (FSC-A vs. FSC-H), viable cells (FSC-A vs. Aqua live/dead), and CD4⁺ cells (FSC-A vs. CD4-APC-Cy7). CD4⁺ T cells were analyzed for CD107a (FSC-A vs. CD107a-FITC), CD154 (FSC-A vs. CD154-APC), IL-2 (FSC-A vs. PECy7), IFNγ (FSC-A vs. IFNγ-PE), and TNF (FSC-A vs. TNF-Pacific Blue) expression.

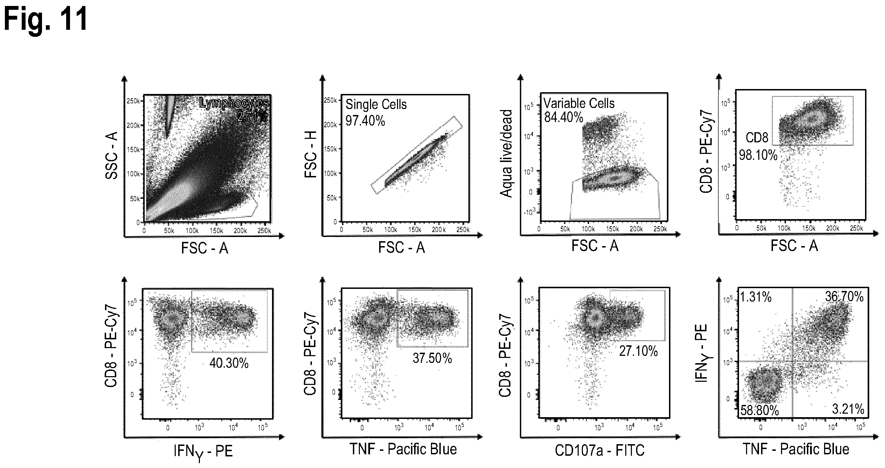

- Fig. 11:: Gating strategy for ICS evaluation of aAPC primed CD8⁺ T cells. Exemplary sample showing the gating strategy for intracellular cytokine stainings (ICS) of artificial antigen presenting cell (aAPC) primed CD8⁺ T cells. The first gate identifies the lymphocytes (FSC-A vs. SSC-A), which are further gated for single cells (FSC-A vs. FSC-H), viable cells (FSC-A vs. Aqua live/dead), and CD8⁺ cells (FSC-A vs. CD8-PE-Cy7). CD8⁺ T cells were analyzed for IFNγ (IFNγ-PE vs. CD8-PE-Cy7), TNF (TNF-Pacific Blue vs. CD8-PE-Cy7), CD107a (CD107a-FITC vs. CD8-PE-Cy7), and TNF/IFNγ (TNF-Pacific Blue vs. IFNγ-PE) expression.

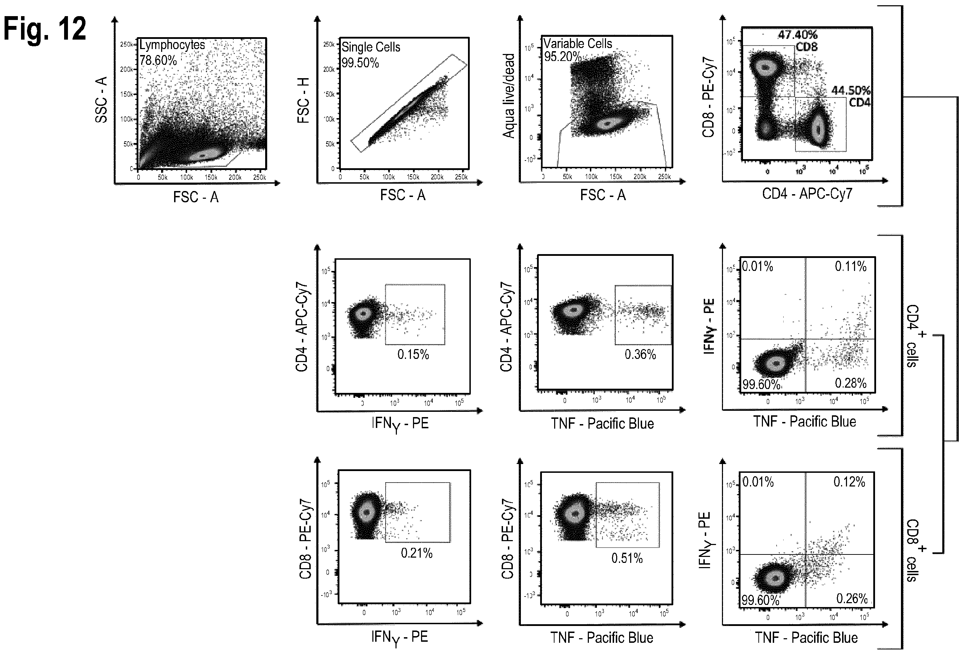

- Fig. 12:: Gating strategy for ICS evaluation of peptide-specific CD4⁺ and CD8⁺ T cells from a FL-HCC patient vaccinated with a personalized DNAJB1-PRKACA-derived peptide vaccine. Exemplary sample showing the gating strategy for intracellular cytokine stainings (ICS) of peptide-specific CD4⁺ and CD8⁺ T cells from a FLHCC patient vaccinated with a personalized DNAJB1-PRKACA-derived peptide vaccine. The first gate identifies the lymphocytes (FSC-A vs. SSC-A), which are further gated for single cells (FSC-A vs. FSC-H), and viable cells (FSC-A vs. Aqua live/dead). The CD4⁺ and CD8⁺ T cells (CD4-APC-Cy7 vs. CD8-PE-Cy7) were analyzed separately for cytokine production. CD8⁺ T cells were analyzed for IFNγ (IFNγ-PE vs. CD8-PE-Cy7), TNF (TNF-Pacific Blue vs. CD8-PE-Cy7), and TNF/IFNγ (TNF-Pacific Blue vs. IFNγ-PE) expression, CD4⁺ T cells for IFNγ (IFNγ-PE vs. CD4-APC-Cy7), TNF (TNF-Pacific Blue vs. CD4-APC-Cy7), and TNF/IFNγ (TNF-Pacific Blue vs. IFNγ-PE) expression.

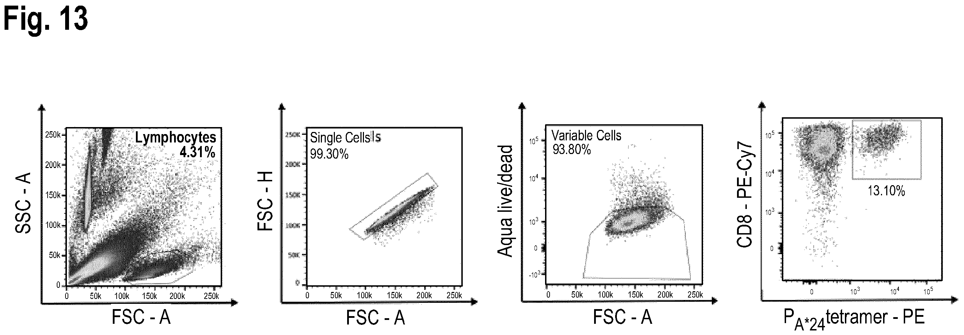

- Fig. 13:: Gating strategy for evaluation of P_{A*24}-tetramer staining of CD8⁺ T cells. Exemplary sample showing the gating strategy for evaluation of P_{A*24}-tetramer staining of CD8⁺ T cells after antigen presenting cell (aAPC)-based priming. The first gate identifies the lymphocytes (FSC-A vs. SSC-A), which are further gated for single cells (FSC-A vs. FSC-H), viable cells (FSC-A vs. Aqua live/dead), and analyzed for CD8⁺/P_{A*24}-tetramer specific cells (P_{A*24} tetramer-PE vs. CD8-PE-Cy7).

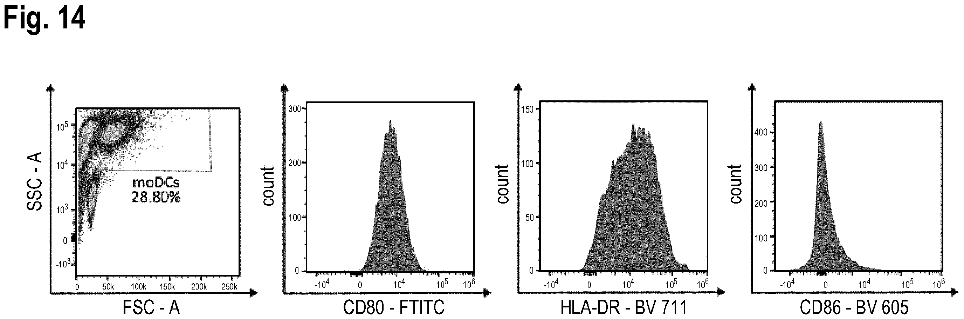

- Fig. 14:: Gating strategy for evaluation of matured moDC. Exemplary sample showing the gating strategy for evaluation of mature monocyte-derived dendritic cells (moDC). The first gate identifies the cells (FSC-A vs. SSC-A), which are further analyzed for CD80 (CD80-FITC vs. count), HLA-DR (HLA-DR-BV 711 vs. count), and CD86 (CD86-BV 605 vs. count) surface expression.

### 1. Introduction

T-cell-based immunotherapies, comprising immune checkpoint inhibitors (ICI), CAR-T cells, adoptive T-cell transfer, and vaccination strategies achieved a breakthrough in the treatment of malignant disease. However, these therapies, which rely on the rejection of cancer cells through recognition of tumor antigens and T cell-mediated cytotoxicity, are still only available and effective in small subsets of cancer patients and single tumor entities. One main reason in particular for the development of antigen-specific immunotherapies is the lack of suitable target structures that show natural, highfrequency, and tumor-exclusive presentation on the cell surface of tumor cells and are recognized by the immune system. Tumor antigens are represented by HLA-independent surface molecules or by HLA class I and II-presented T-cell epitopes, originating from intracellular proteins. In terms of the latter, neoepitopes, arising from tumor-specific mutations have been identified in recent years as main specificity of anti-cancer T-cell responses induced by ICIs and thus suggested as prime candidates for T-cell-based immunotherapy approaches. In line, response to ICI is correlated with high tumor somatic mutational burden and neoepitope-based immunotherapies showed first promising results in individual tumor patients. However, patient/tumor-specificity and intratumoral heterogeneity of somatic mutations as well as the limited number of somatic mutations translated, processed, and presented as HLA-restricted neoepitope on the tumor cells restrict the broad applicability of these antigens in particular in low-mutational burden entities. Recently, fusion transcripts, whose products often represent clonal oncogenic drivers, were identified as a novel source of highly immunogenic neoepitopes. T-cell responses against such fusion protein-derived neoepitopes were detected in patients receiving ICI and correlated with treatment response.

The *DNAJB1-PRKACA* fusion transcript links exon 1 of the DnaJ homolog subfamily B member 1 gene (*DNAJB1*) to the cAMP-dependent protein kinase catalytic subunit alpha gene (*PRKACA*)*.* This fusion transcript was first identified in fibrolamellar hepatocellular carcinoma (FL-HCC) a rare but lethal tumor disease, lacking established treatment options beside surgical resection, which typically affects children and adolescents with no history of primary liver disease. In addition, the *DNAJB1-PRKACA* fusion could be detected in multiple other gastroenterological tumor diseases. In FL-HCC the *DNAJB1-PRKACA* fusion transcript is detectable in 100% of patients and was identified as the oncogenic driver in tumor pathogenesis, indicating expression of the fusion transcript in all tumor cells. The inventors here could demonstrate that the *DNAJB1-PRKACA* fusion transcript represents a prime source for broadly applicable neoepitopes and provided first evidence for their application in immunotherapy approaches in an FL-HCC patient.

### 2 Results

### 2.1 The DNAJB1-PRKACA fusion gene is a source of HLA class I and II neoantigens and induces DNAJB1-PRKACA-specific CD4⁺T cells

A *in silico* prediction workflow using the algorithm NetMHCIIpan identified nine binding cores of nine amino acid (AA) length for a total of 1290 different HLA class II alleles, within the 24 AA fusion region of the DNAJB1-PRKACA protein (Fig. 1a,b). 83.49% of these alleles represent HLA-DP combinations, 11.55% and 4.96% were HLA-DQ or HLA-DR combinations, respectively. The core sequence RYGEEVKEF (SEQ ID NO: 5), located directly in the middle of the fusion transcript (5 AAs on exon 1 (*DNAJB1*)*,* 4 AAs on exon 2 (*PRKACA))* provides the highest amount of predicted HLA class II alleles (60.52% of possible allele/binding core-combinations). For HLA class I 13 DNAJB1-PRKACA-derived HLA ligands were identified for the 20 most frequent HLA class I allotypes of the European population, including peptides comprising SEQ ID NOs: 3, 4, and 5, using a prediction workflow combining the algorithms SYFPEITHI and NetMHCpan (Fig. 1c). Of note, the HLA class II-binding core RYGEEVKEF (SEQ ID NO: 5) was also predicted as HLA class I ligand binding to the alleles HLA-A*24:02, C*04:01, C*06:02, and C*07:02.

Natural presentation of DNAJB1-PRKACA-derived HLA-presented peptides was shown by liquid chromatography-coupled tandem mass spectrometry (LC-MS/MS) of differentiated and matured monocyte-derived dendritic cells (moDCs; Fig 1d) from healthy volunteers (HV) loaded with the 22 AA peptide
KREIFDRYGEEVKEFLAKAKED (P_{II-1}) (SEQ ID NO: 1), spanning the fusion region of the DNAJB1-PRKACA protein. The mass spectrometric (MS) identified fragment ion spectra of the experimentally eluted P_{II-1} was validated using the isotope-labeled synthetic peptide (Fig. 1e). P_{II-1} and 12 shorter length variants identified by MS were predicted to bind to the HLA-DP allele DPA1*01:03-DPB1*05:01 of the respective HV with the best NetMHCIIpan binding rank of 0.74 for the binding core RYGEEVKEF (SEQ ID NO: 5), including a peptide comprising SEQ ID NO: 1 (Fig1b). *De novo* priming of CD4⁺ T cells from HVs with P_{II-1} loaded mature moDCs induced multifunctional P_{II-1}-specific CD4⁺ T cells with expression of CD107a, CD154, II-2, IFNγ, and TNF upon P_{II-1}-pulsing (Fig. 1f).

### 2.2 Characterization of P_{A}*₂₄-specific CD8⁺ T cells and single-cell TCR sequencing

Refolding of the DNAJB1-PRKACA protein fusion-derived ligand RYGEEVKEF (SEQ ID NO: 5) (P_{A}*₂₄, SYFPEITHI score of 74.19% and NetMHCpan rank of 0.018) was conducted to a HLA-A*24-P_{A*24}-monomer. Artificial antigen presenting cell (aAPC)-priming of CD8⁺ T cells of HVs using this monomer induced P_{A*24}-specific CD8⁺ T cells, with frequencies of up to 15.74% peptide-specific T cells (mean 4.97%; Fig. 2a,b). P_{A*24}-specific CD8⁺ T cells showed a polyfunctional phenotype reflected by IFNγ, TNF, and CD107a production/expression (Fig. 2c) and specifically lysed P_{A*24}-loaded autologous CD8⁻ cells *in vitro,* with up to 82.39% lysis of target cells in comparison to unspecific effector cells at various effector to target ratios (Fig. 2d). Single-cell T-cell receptor (TCR) sequencing of flow cytometry-based bulk sorted P_{A*24}-specific CD8⁺ T cells from two HVs (HV1, HV2) after *in vitro* aAPC-priming showed a high clonality of one dominant TCR clone for HV2 and two clones for HV1 (Fig. 3a,b). There was no overlap of the V(D)J gene sequence of the three TCR-clones, but a high similarity was observed in terms of the physiochemical properties of the AAs from the CDR3-α/-β sequences between the TCR-clones, showing opposing characteristics regarding hydrophilicity and chemical grouping in comparison to their target peptide P_{A*24} (Fig. 2e, Fig. 3c).

### 2.3 Doxycycline-inducible DNAJB1-PRKACA fusion gene expression leads to natural processing and presentation of HLA class I and HLA class II DNAJB1-PRKACA specific ligands

Transduction of three HLA class I expressing hepatocellular carcinoma (HCC) cell lines HLE, SMMC-7721, and HepG2 with a Doxycycline (Dox)-inducible plasmid, containing the *DNAJB1-PRKACA* fusion gene under the control of a tetracycline-responsive promoter, enabled the specific expression of the DNAJB1-PRKACA fusion protein (Fig. 4a,b, Fig. 5a). MS-based immunopeptidome analyses of the HCC cell lines after *DNAJB1-PRKACA* fusion gene expression revealed up to 3688 HLA class I ligands, including peptides comprising SEQ ID NOs: 6 - 63 (mean 2787; Fig. 4c). 20 unique HLA class I ligands derived from the DNAJB1-PRKACA fusion protein were identified with two ligands spanning the fusion region (Fig. 4d). The two naturally processed and presented DNAJB1-PRKACA-derived neoepitopes EIFDRYGEEV (SEQ ID NO: 3) (P_{A*68/A*02}) identified on SMMC-7721, and IFDRYGEEV (SEQ ID NO: 4) (P_{C*04/C*05}) identified on HepG2, were predicted to bind to the HLA allotype A*68:02 and C*04:01 of the respective cell line, respectively, and could be validated by comparative spectra analysis using synthetic peptides (Fig. 1c, Fig. 4e). To investigate the processing and presentation of HLA class II peptides derived from the DNAJB1-PRKACA fusion protein mature moDCs of three HVs were incubated with lysate of the HLE cell line after activation of *DNAJB1-PRKACA* fusion gene expression (Fig. 5b). MS-based immunopeptidome analysis of these moDCs revealed up to 8293 HLA class II peptides (mean 5956; Fig. 4f), and 13 unique peptides derived from the DNAJB1-PRKACA fusion protein were identified, with one peptide EVKEFLAKAKEDFLKK (SEQ ID NO: 2) (P_{II-2}) spanning the fusion region (Fig. 4g). The DNAJB1-PRKACA-derived neoepitope P_{II-2} was predicted to bind to the HLA allele DRB1*13:02 of the HV (Fig.1b). The experimental fragment ion spectrum of the P_{II-2} peptide was validated with an isotopically-labeled synthetic peptide (Fig. 4h). Of note, no HLA class I or HLA class II ligands from either of the two fusion proteins were identified in the respective negative controls.

### 2.4 Personalized DNAJB1-PRKACA-derived peptide vaccine induced long-lasting DNAJB1-PRKACA-specific immune response and showed favorable clinical outcome in a FL-HCC patient

A personalized DNAJB1-PRKACA-derived peptide vaccine was formulated for a young patient with histologically confirmed FL-HCC (FL-HCC01) who experienced multiple tumor relapses after early liver transplant (LTx) due to unresectable FL-HCC not responsive to chemotherapy (Fig. 6a and Fig. 7a). The mTOR inhibitor everoli-mus was applied for post-transplant immunosuppression. Poly ADP ribose polymerase (PARP) inhibition was initiated based on alterations in the DNA Damage Response (DDR) pathway (ATM and CHEK2, germline variant, BRCA2 und BAP1 somatic deletion), not achieving durable remission. Recurrent tumor manifestations were resected or treated with radiotherapy. Based on the *DNAJB1-PRKACA* fusion gene confirmed by sanger sequencing the personalized vaccine was composed of three short allotype-matching HLA class I ligands (EIFDRYGEEV (SEQ ID NO: 3) (P_{A*68/A*02}), EEVKEFLAKA (SEQ ID NO: 120) (P_{B*44}), and IFDRYGEEV (SEQ ID NO: 4) (P_{C*04/C*05})), together with the long peptide KREIFDRYGEEVKEFLAKAKED (SEQ ID NO: 1) (P_{II-1}) predicted to bind to the HLA-DP allotype DPA1*01:03-DPB1*06:01 of FL-HCC01. The vaccine was adjuvanted with the novel toll-like receptor (TLR) 1/2 agonist XS15 emulsified in Montanide^{™} ISA51 VG, which endorse activation and maturation of antigen presenting cells and prevent vaccine peptides from immediate degradation, enabling the induction of an effective and potent T-cell response (Fig. 7b). The personalized DNAJB1-PRKACA-derived peptide vaccine was applied twice within a 6 week interval. Induction of a profound T-cell response targeting the P_{II-1} peptide was observed six weeks after the second vaccination, analyzed by IFNγ enzyme-linked immunospot (ELISPOT) assay after *in vitro* stimulation with the vaccine cocktail peptides (1 mean spot count prior to vaccination versus 872 post second vaccination; Fig. 6b,c and Fig. 7c). In addition a weaker T-cell response was detected targeting the HLA class I peptide P_{B*44} (0 mean spot count prior to vaccination versus 56 post second vaccination). Longitudinal IFNγ ELISPOT assays showed persistence of P_{II-1}-specific T cells over time with an even increased intensity (1088 mean spot count) 45 weeks after the second vaccination (Fig. 6c and Fig. 7c). Flow cytometry-based characterization of the P_{B*44} and P_{II-1}-directed immune response revealed T helper 1 (Th1) phenotype CD4⁺ T cells with specific expression of IFNγ and TNF. No CD8⁺ T-cell response against P_{B*44} was observed. Of note, no disease relapse was observed in FL-HCC01 until the latest examination 15 months after the second vaccination, suggesting first sings of clinical efficacy of vaccine-induced DNAJB1-PRKACA-specific T-cell responses.

### 2.5 Single-cell RNA sequencing showed TCR clonality in vaccine-induced P_{II-1} specific CD4⁺ T cells

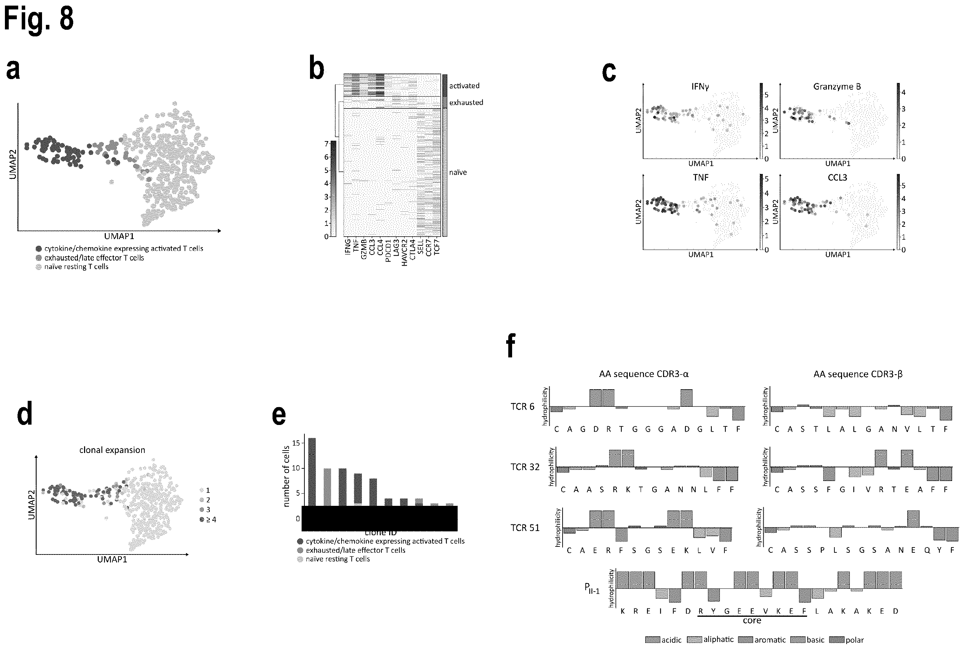

Unsupervised clustering of 10× Genomics' single-cell RNA sequencing data from vaccine-induced HLA-DPA1*01:03-DPB1*06:01-P_{II-1} reactive CD4⁺ T cells after *in vitro* expansion showed three defined T-cell clusters: (i) cytokine and chemokine expressing activated T cells defined by high expression of *IFNG, TNF, GZMB* (encoding granzyme B), *CCL3,* and *CCL4* (Fig. 5a,b,c), (ii) T cells exhibiting an exhausted or late effector profile with expression of *PDCD1, LAG3, HAVCR2,* and *CTLA4* (Fig. 8a,b and Fig. 9a), and (iii) naive resting T cells defined by expression of *SELL* (encoding CD62L), *CCR7,* and *TCF7*(Fig*.* 8a,b and Fig. 9b). High TCR-clonality was observed in the activated T-cell cluster, with 74.2% of cells assigned to large clones (clonality ≥4) compared to the naive resting (1.3%) or exhausted T-cell cluster (31.4%) (Fig. 8d and Fig. 9c). In total ten defined TCR-clones were identified of which eight were predominantly assigned to the activated T-cell cluster. High similarity of the physiochemical properties was observed between the TCR-clones for AAs from the CDR3-α/-β sequences, showing opposing characteristics with regard to hydrophilicity and chemical grouping in comparison to their target peptide P_{II-1} (Fig. 8e,f, Fig. 9d).

### 3. Material and Methods

### 3.1 Patients and blood samples

Peripheral blood mononuclear cells (PBMCs) from the FL-HCC patient as well as PBMCs from healthy volunteers (HVs) were isolated by density gradient centrifugation and stored at -80°C until further use for subsequent T cell-based assays. Informed consent was obtained in accordance with the Declaration of Helsinki protocol. The study was performed according to the guidelines of the local ethics committees (713/2018B02, 406/2019BO2). HLA typing of samples was carried out by the Department of Hematology and Oncology, Tübingen, Germany.

### 3.2 Personalized peptide vaccine

The personalized vaccine developed and produced by the Good Manufacturing Practices (GMP) Peptide Laboratory of the Department of Immunology, University Tübingen, is a peptide-based vaccine containing 4 DNAJB1-PRKACA-derived peptides (SEQ ID NOs: 1, 3, 4, 120) and the adjuvant lipopeptide synthetic TLR1/2 ligand XS15 (manufactured by Bachem AG, Bubendorf, Switzerland) emulsified in Montanide^{™} ISA51 VG (manufactured by Seppic, Paris, France). Vaccine peptides (250 µg/peptide) and XS15 (50 µg) are prepared as a water-oil emulsion 1:1 with Montanide^{™} ISA51 VG to yield an injectable volume of 500 µL. The patient received subcutaneous injection of the personalized vaccine at the lower abdomen. Personalized vaccination was performed under the protocol NCT05014607.

### 3.3 Detection of DNAJB1-PRKACA transcript and sequencing

RNA was extracted from macrodissected 5 µm paraffin sections using the Maxwell^{®} RSC RNA FFPE Kit and the Maxwell^{®} RSC Instrument (Promega, Madison, WI, USA) according to the manufacturer's instructions. Reverse transcription of RNA and polymerase chain reaction (PCR) of the *DNAJB1-PRKACA* breakpoint region (forward primer 5'-GTTCAAGGAGATCGCTGAGG-3' (SEQ ID NO: 122), reverse primer 5'-TTCCCGGTCTCCTTGTGTTT-3' (SEQ ID NO: 123) was performed using the QIAGEN OneStep RT-PCR Kit according to the manufacturer's instructions (Qiagen, Hilden, Germany). To visualize the detection of the DNAJB1-PRKACA fusion, the PCR product was run on an agarose gel. For sequencing the PCR product was purified (AMPure, Beckman Coulter, Brea, CA, USA) and aliquots were used for the sequencing reaction with 1 µM of the forward or reverse primer and 2 µl of GenomeLab DTCS-Quick Start Master Mix (Beckman Coulter, Brea, CA, USA) in a final volume of 10 µl according to the manufacturers protocol. Sequencing reactions were purified (CleanSEQ, Beckman Coulter, Brea, CA, USA) and analyzed in a GenomeLab GeXP Genetic Analysis System and evaluated by the GenomeLab GeXP software (Beckman Coulter, Brea, CA, USA).

### 3.4 In silico DNAJB1-PRKACA ligand prediction

HLA class I DNAJB1-PRKACA ligand prediction was performed for all possible 8-12 amino acid long peptide sequences spanning the fusion region, using SYFPEITHI 1.0 and NetMHCpan 4.1 for the 20 most frequent HLA class I allotypes in the European population (tools.iedb.org). HLA class II DNAJB1-PRKACA ligand prediction was performed for all possible 15 amino acid long peptide sequences spanning the fusion region, using NetMHCIIpan 4.0 with all listed allele combinations possible.

### 3.5 Quantification of HLA surface expression

HLA surface expression of HCC cell lines was analyzed using the QIFIKIT bead-based quantitative flow cytometric assay (Dako, K0078) according to manufacturer's instructions as described before. In brief, samples were stained with the pan-HLA class I-specific monoclonal antibody (mAb) W6/32 (produced in-house, or IgG isotype control (BioLegend, 400202). Flow cytometric analysis was performed on a FACSCanto II Analyzer (BD).

### 3.6 Doxycycline-inducible DNAJB1-PRKACA fusion gene expression in HCC cell lines

The HCC cell lines HLE, SMMC-7721 and HepG2 were cultivated in Gibco Dulbecco's Modified Eagle Medium supplemented with 10% fetal calf serum (FCS), penicillin, streptomycin (all from Merck) and plasmocin (Invivogen) at 37°C and 5% CO2 in a humidified atmosphere. The DNAJB1-PRKACA coding sequence was synthetized by ThermoFisher, cloned into the pENTR^{™} and transferred by directional TOPO cloning (pENTR^{™}/D-TOPO^{™} cloning kit, Invitrogen) into the plnducer20 (Addgene #44012) destination vector. Lentiviral particles were produced in HEK293T cells by calciumphosphate transfection of the helper plasmids psPAX2, pMD2.G and either the empty or the DNAJB1-PRKACA coding plnducer20. The transduced HCC cell lines were selected with G418 (Invivogen) for at least two weeks. To induce the expression of DNAJB1-PRKACA, the transduced HCC cell lines were treated with 1 µg/ml Doxycycline (Dox) for 24 h (AppliChem). To validate the DNAJB1-PRKACA expression, cells were lysed in lysis buffer (50 mM Tris-HCI pH 7.4, 150 mM NaCl, 1% Triton X-100, 50 mM NaF, 10 mM Na₄P₂O₇, 10 mM Na₄V₂O₇ and complete protease inhibitor cocktail (Roche)). SDS-PAGE and immunoblotting were performed as described previously. For immunoblotting the following primary antibodies were used: anti-PKAa cat (Santa Cruz, clone A-2), anti-GAPDH (Cell Signaling, clone D16H11) and anti-Tubulin (Merck, clone DM1A). HRP-coupled goat anti-rabbit or goat anti-mouse (both Jackson ImmunoResearch) secondary antibodies were used for visualization.

### 3.7 Isolation of HLA ligands

HLA class I and HLA class II molecules were isolated by standard immunoaffinity purification using the pan-HLA class I-specific mAb W6/32, the pan-HLA class II-specific mAb Tü-39, and the HLA-DR-specific mAb L243 (all produced in-house) to extract HLA ligands.

### 3.8 Analysis of HLA ligands by liquid chromatoqraphy-coupled tandem mass spectrometry (LC-MS/MS)

Peptide samples were separated by reversed-phase liquid chromatography (nanoUHPLC, UltiMate 3000 RSLCnano, Thermo Fisher, Waltham, Massachusetts, USA) and subsequently analyzed in an on-line coupled Orbitrap Fusion Lumos mass spectrometer (Thermo Fisher, Waltham, Massachusetts, USA). Samples were analyzed in three technical replicates. Sample volumes of 5 µL with shares of 20% were injected onto a 75 µm × 2 cm trapping column (Thermo Fisher, Waltham, Massachusetts, USA) at 4 µL/min for 5.75 min. Peptide separation was subsequently performed at 50 °C and a flow rate of 300 nL/min on a 50 µm × 25 cm separation column (PepMap C18, Thermo Fisher, Waltham, Massachusetts, USA) applying a gradient ranging from 2.4 to 32.0% of ACN over the course of 90 min. Eluting peptides were ionized by nanospray ionization and analyzed in the mass spectrometer implementing a top speed (3 s) HCD (Higher-energy C-trap dissociation) method generating fragment spectra with a resolution of 30,000, a mass range limited to 235-1151 m/z, and positive charge states 2-5 selected for fragmentation.

### 3.9 Data processing

Data processing was performed as described previously. The Proteome Discoverer (v1.3, Thermo Fisher) was used to integrate the search results of the SequestHT search engine (University of Washington) against the human proteome (Swiss-Prot database, 20 279 reviewed protein sequences, September 27th 2013) accompanied with the complete sequence of the DNAJB1-PRKACA fusion protein. Precursor mass tolerance was set to 5 ppm and fragment mass tolerance was set to 0.02 Da. Oxidized methionine was allowed as dynamic modification. The false discovery rate (FDR, estimated by the Percolator algorithm 2.04) was limited to 5% for HLA class I- and 1% for HLA class II-presented peptides. HLA class I annotation was performed using SYFPEITHI 1.0 and NetMHCpan 4.1.

### 3.10 Spectrum validation

Spectrum validation of the experimentally eluted peptides was performed by computing the similarity of the spectra with corresponding synthetic peptides measured in a complex matrix. The spectral correlation was calculated between the MS/MS spectra of the eluted and the synthetic peptide.

### 3.11 Amplification of peptide-specific T cells and IFNγ ELISPOT assay

PBMCs were pulsed either with 1 µg/ml or with 5 µg/ml of HLA class I or HLA class II peptide, respectively. Irrelevant peptides with the respective HLA restrictions were used as negative control: YLLPAIVHI (SEQ ID: 124) for HLA-A*02 (source protein: DDX5_HUMAN), and ETVITVDTKAAGKGK (SEQ ID NO: 125) for HLA class II (source protein: FLNA_HUMAN). Cells were cultured for 12 days adding 20 U/ml IL-2 (Novartis, Basel, Switzerland) on days 2, 5, and 7. Peptide-stimulated PBMCs were analyzed by IFNγ enzyme-linked immunospot (ELISPOT) assay on day 12. Spots were counted using an ImmunoSpot S6 analyzer (CTL, Cleveland, OH, USA) and T-cell responses were considered positive if > 10 spots/500,000 cells were counted, and the mean spot count was at least three-fold higher than the mean spot count of the negative control.

### 3.12 Refolding

Biotinylated HLA:peptide complexes were manufactured as described previously and tetramerized using PE-conjugated streptavidin (Invitrogen) at a 4:1 molar ratio.

### 3.13 Induction of peptide-specific CD8⁺ T cells with aAPCs

Priming of peptide-specific cytotoxic T lymphocytes was conducted using artificial antigen-presenting cells (aAPCs) as described previously. In detail, 800,000 streptavidin-coated microspheres (Bangs Laboratories, Fishers, Indiana, USA) were loaded with 200 ng biotinylated HLA:peptide monomer and 600 ng biotinylated anti-human CD28 monoclonal antibody (clone 9.3, in-house production). CD8⁺ T cells were cultured with 4.8 U/µl IL-2 (R&D Systems, Minneapolis, MN, USA) and 1.25 ng/ml IL-7 (PromoKine, Heidelberg, Germany). Weekly stimulation with aAPCs (200,000 aAPCs per 1 × 10⁶ CD8⁺ T cells) and 5 ng/ml IL-12 (PromoKine) was performed for four cycles.

### 3.14 Cytokine and tetramer staining

Functionality of peptide-specific CD4⁺ and CD8⁺ T cells was analyzed by intracellular cytokine staining (ICS) as described previously. Cells were pulsed with 10 µg/ml of respective peptide and incubated with 10 µg/ml Brefeldin A (Sigma-Aldrich, Saint Louis, MO, USA) and 10 µg/ml GolgiStop (BD, Franklin Lakes, NJ, USA) for 12-16 h. Staining was performed using Cytofix/Cytoperm (BD), APC/Cy7 anti-human CD4 (1:100 dilution, BioLegend, Cat# 300518, RRID: AB_314086), PE/Cy7 anti-human CD8 (1:400 dilution, Beckman Coulter, Cat# 737661, RRID: AB_1575980), Pacific Blue anti-human tumor necrosis factor (TNF, 1:120 dilution, BioLegend, Cat# 502920, RRID: AB_528965), FITC anti-human CD107a (1:100 dilution, BioLegend, Cat# 328606, RRID: AB_1186036), APC anti-human IL-2 (1:40 dilution, BioLegend, Cat# 500309, RRID: AB_315096), and PE anti-human IFNy monoclonal antibodies (1:200 dilution, BioLegend, Cat# 506507, RRID: AB_315440). PMA and ionomycin (Sigma-Aldrich) served as positive control. Negative control peptides with matching HLA restrictions were used: YLLPAIVHI (SEQ ID NO: 124) for HLA-A*02 (source protein: DDX5_HUMAN), KYPENFFLL (SEQ ID NO: 126) for HLA-A*24 (source protein: PP1G_HUMAN), EEFGRAFSF (SEQ ID NO: 127) for HLA-B*44 (source protein: HLA-DP_HUMAN), and ETVITVDTKAAGKGK (SEQ ID NO: 125) for HLA class II (source protein: FLNA_HUMAN). Gating strategies applied for the analyses of flow cytometry-acquired data are provided in Fig. 10, 11 and 12.

The frequency of peptide-specific CD8⁺ T cells after aAPC-based priming was determined by PE-Cy7 anti-human CD8 monoclonal antibody and HLA:peptide tetramer-PE staining. Cells of the same donor primed with an irrelevant control peptide TYSEKTTLF (SEQ ID NO: 128) (source protein: MUC16_HUMAN) and stained with the tetramer containing the test peptide were used as negative control. The priming was considered successful if the frequency of peptide-specific CD8⁺ T cells was ≥ 0.1% of CD8⁺ T cells within the viable single cell population and at least three-fold higher than the frequency of peptide-specific CD8⁺ T cells in the negative control. The same evaluation criteria were applied for ICS results. Samples were analyzed on a FACS Canto II cytometer (BD). The gating strategy applied for tetramer staining analysis of flow cytometry-acquired data is provided in Supplementary Fig. 13.

### 3.15 Cytotoxicity assays

Peptide-specific CD8⁺ T cells were analyzed for their capacity to induce peptide-specific target cell lysis in the flow cytometry-based VITAL assay. Autologous CD8⁻ target cells were either loaded with the P_{A*24} peptide or the negative peptide KYPENFFLL (SEQ ID NO: 126) (source protein: PP1G_HUMAN) and labeled with CFSE or FarRed (life technologies, Carlsbad, CA, USA), respectively. The P_{A*24}-specific effector cells were added in the indicated effector-to-target ratios. Specific lysis of peptide-loaded CD8⁻ target cells was calculated relative to control targets.

### 3.16 Induction of peptide-specific CD4⁺ T cells with peptide loaded moDCs

For the differentiation of monocyte-derived dendritic cells (moDCs) CD14⁺ cells were isolated from PBMC using magnetic-activated cell-sorting (MACS; Miltenyi, Bergisch Gladbach, Germany), and subsequently cultivated in X-VIVO^{™} 15 Serum-free Hematopoietic Cell Medium supplemented with, penicillin, streptomycin, GM-CSF, and IL-4, at 37°C and 5% CO₂ in a humidified atmosphere for seven days. Differentiated moDCs were maturated by adding LPS to the cell culture medium for 24 h and checked for cell surface expression of CD80-FITC (1:40 dilution, Biolegend, Cat# 305206), HLA-DR-BV 711 (1:100 dilution, Biolegend, Cat# 307644), and CD86-BV 605 (1:400 dilution, Biolegend, Cat# 374214), the gating strategy applied for the analysis of flow cytometry-acquired data is provided in Fig. 14. Mature moDCs were incubated with the P_{II-1} peptide for 2 h prior to CD4⁺ T-cell stimulation. CD4⁺ cells were isolated from PBMC of the same healthy volunteer (HV) using magnetic-activated cell-sorting (MACS; Miltenyi, Bergisch Gladbach, Germany), and subsequently cultivated in X-VIVO^{™} 15 Serum-free Hematopoietic Cell Medium supplemented with, penicillin, streptomycin, IL-2, and IL-7, at 37°C and 5% CO₂ in a humidified atmosphere. Cultured CD4⁺ cells were stimulated weekly, for a total of four weeks, with peptide-loaded mature moDCs and IL-12. The functionality of peptide-specific CD4⁺ T cells was analyzed by ICS.

### 3.17 Antigen-loading of mature moDCs

For the generation of tumor lysate, HCC cell lines transduced with the empty or the DNAJB1-PRKACA coding plasmid were treated with IFNyand Dox for 24 h. The treated cells were harvested, washed with PBS, subjected to five freeze-thaw cycles, irradiated with 30 Gy, and sonicated for 2 min. The clear supernatant was then added to the cell culture medium of maturated moDCs for 24 h, and the antigen-loaded mature moDCs were subsequently harvested for HLA immunoprecipitation.

### 3.18 Software and statistical analysis

The population coverage of HLA allotypes was calculated by the IEDB population coverage tool (www.iedb.org). All Fig. s and statistical analyses were generated using GraphPad Prism 9.2.0 (GraphPad Software). *P* values of < 0.05 were considered statistically significant.

### 3.19 Single cell immune profiling

Peptide-specific CD8⁺ T cells or bulk CD4⁺ T cells were FACS sorted, counted and washed in 0.04% BSA/PBS according to the 10x Genomics cell preparation protocol. Single cells were partitioned into Gel Beads-in-Emulsion (GEMs) together with 10x barcoded Gel Beads and reverse transcriptase enzymatic reaction using the Chromium Controller instrument (10x Genomics, Pleasanton, California, USA). Single cell gene expression libraries and single cell T-cell receptor (VDJ) libraries were then prepared using the Chromium Next GEM Single Cell 5'Kit v2 (10x Genomics, Pleasanton, California, USA) and the Chromium Single Cell Human TCR Amplification Kit (10x Genomics, Pleasanton, California, USA) according to the manufacturer's instructions. Libraries were pooled and sequenced on a NextSeq 550 (Illumina, San Diego, California, USA) at 28,806, 138,975 and 3519 mean reads per cell, respectively. Samples were demultiplexed using bcl2fastq version 2.20.0.422 (Illumina, San Diego, California, USA). Barcode processing, alignment, VDJ annotation, and single-cell 5'gene counting were performed using Cell Ranger Software version 6.0.1 (10x Genomics, Pleasanton, California, USA). Further data processing, visualization, and analysis were done using scanpy and scirpy for each sample separately. Cells with unique gene counts < 200 and without vdj sequence associated, as well as cells with > 10% of mitochondrial genes, were removed from the analysis, keeping 474 cells (FL-HCC01), 115 cells (HV1) and 3338 cells (HV2), respectively. Data was log-normalized to a scale factor of 10,000. Only highly variable genes were considered for linear dimensional reduction and were defined by a minimum

## Claims

1. A peptide comprising an amino acid sequence selected from the group consisting of SEQ ID NO: 1 to SEQ ID NO: 5, and variant sequences thereof which are at least 88% homologous to SEQ ID NO: 1 to SEQ ID NO: 5, and wherein said variant sequences bind to molecule(s) of the major histocompatibility complex (MHC) and/or induce T-cells cross-reacting with said variant sequences; and a pharmaceutical acceptable salt thereof, wherein said peptide is not a full-length polypeptide.

2. The peptide according to claim 1, wherein said peptide has the ability to bind to an MHC class-I or -II molecule, and wherein said peptide, when bound to said MHC, is capable of being recognized by CD4 and/or CD8 T-cells, preferably the amino acid sequence of said peptide comprises a continuous stretch of amino acids according to any one of SEQ ID NO: 1 to SEQ ID NO: 5.

3. An antibody, in particular a soluble or membrane-bound antibody, further preferably a monoclonal antibody or fragment thereof, that specifically recognizes the peptide or variant thereof according to claim 1 or 2, preferably the peptide or variant thereof according to claim 1 or 2 when bound to an MHC molecule.

4. A T-cell receptor, preferably soluble or membrane-bound, or a fragment thereof, that is reactive with an HLA ligand, wherein said ligand is the peptide or variant thereof according to claim 1 or 2, preferably the peptide or variant thereof according to claim 1 or 2 when bound to an MHC molecule.

5. An antigen binding protein which specifically binds at least one DNAJB1-PRKACA antigenic peptide in complex with a major histocompatibility complex (MHC) molecule, wherein the antigen-binding protein comprises a first polypeptide chain which comprises a first variable domain that comprises at least one complementary determining region (CDR), CDR3α, and a second polypeptide chain which comprises at a second variable domain that comprises at least one CDR, CDR3β, wherein
a) CDR3α comprises or consists of the amino acid sequence SEQ ID NO: 6, CDR3β comprises or consists of the amino acid sequence SEQ ID NO: 36, and wherein the DNAJB1-PRKACA antigenic peptide comprises or consists of SEQ ID NO: 1; or
b) CDR3α comprises or consists of the amino acid sequence SEQ ID NO: 7, CDR3β comprises or consists of the amino acid sequence SEQ ID NO: 37, and wherein the DNAJB1-PRKACA antigenic peptide comprises or consists of SEQ ID NO: 1; or
c) CDR3α comprises or consists of the amino acid sequence SEQ ID NO: 8, CDR3β comprises or consists of the amino acid sequence SEQ ID NO: 38, and wherein the DNAJB1-PRKACA antigenic peptide comprises or consists of SEQ ID NO: 1; or
d) CDR3α comprises or consists of the amino acid sequence SEQ ID NO: 9, CDR3β comprises or consists of the amino acid sequence SEQ ID NO: 39, and wherein the DNAJB1-PRKACA antigenic peptide comprises or consists of SEQ ID NO:1; or
e) CDR3α comprises or consists of the amino acid sequence SEQ ID NO: 10, CDR3β comprises or consists of the amino acid sequence SEQ ID NO: 40, and wherein the DNAJB1-PRKACA antigenic peptide comprises or consists of SEQ ID NO: 1; or
f) CDR3α comprises or consists of the amino acid sequence SEQ ID NO: 11, CDR3β comprises or consists of the amino acid sequence SEQ ID NO: 41, and wherein the DNAJB1-PRKACA antigenic peptide comprises or consists of SEQ ID NO: 1; or
g) CDR3α comprises or consists of the amino acid sequence SEQ ID NO: 12, CDR3β comprises or consists of the amino acid sequence SEQ ID NO: 42, and wherein the DNAJB1-PRKACA antigenic peptide comprises or consists of SEQ ID NO: 1; or
h) CDR3α comprises or consists of the amino acid sequence SEQ ID NO: 13, CDR3β comprises or consists of the amino acid sequence SEQ ID NO: 43, and wherein the DNAJB1-PRKACA antigenic peptide comprises or consists of SEQ ID NO: 1; or
i) CDR3α comprises or consists of the amino acid sequence SEQ ID NO: 14, CDR3β comprises or consists of the amino acid sequence SEQ ID NO: 44, and wherein the DNAJB1-PRKACA antigenic peptide comprises or consists of SEQ ID NO: 1; or
j) CDR3α comprises or consists of the amino acid sequence SEQ ID NO: 15, CDR3β comprises or consists of the amino acid sequence SEQ ID NO: 45, and wherein the DNAJB1-PRKACA antigenic peptide comprises or consists of SEQ ID NO: 1; or
k) CDR3α comprises or consists of the amino acid sequence SEQ ID NO: 32, CDR3β comprises or consists of the amino acid sequence SEQ ID NO: 45, and wherein the DNAJB1-PRKACA antigenic peptide comprises or consists of SEQ ID NO: 1; or
l) CDR3α comprises or consists of the amino acid sequence SEQ ID NO: 16, CDR3β comprises or consists of the amino acid sequence SEQ ID NO: 46, and wherein the DNAJB1-PRKACA antigenic peptide comprises or consists of SEQ ID NO: 1; or
m) CDR3α comprises or consists of the amino acid sequence SEQ ID NO: 17, CDR3β comprises or consists of the amino acid sequence SEQ ID NO: 47, and wherein the DNAJB1-PRKACA antigenic peptide comprises or consists of SEQ ID NO: 1; or
n) CDR3α comprises or consists of the amino acid sequence SEQ ID NO: 33, CDR3β comprises or consists of the amino acid sequence SEQ ID NO: 47, and wherein the DNAJB1-PRKACA antigenic peptide comprises or consists of SEQ ID NO: 1; or
o) CDR3α comprises or consists of the amino acid sequence SEQ ID NO: 18, CDR3β comprises or consists of the amino acid sequence SEQ ID NO: 48, and wherein the DNAJB1-PRKACA antigenic peptide comprises or consists of SEQ ID NO: 1; or
p) CDR3α comprises or consists of the amino acid sequence SEQ ID NO: 19, CDR3β comprises or consists of the amino acid sequence SEQ ID NO: 49, and wherein the DNAJB1-PRKACA antigenic peptide comprises or consists of SEQ ID NO: 5; or
q) CDR3α comprises or consists of the amino acid sequence SEQ ID NO: 19, CDR3β comprises or consists of the amino acid sequence SEQ ID NO: 62, and wherein the DNAJB1-PRKACA antigenic peptide comprises or consists of SEQ ID NO: 5; or
r) CDR3α comprises or consists of the amino acid sequence SEQ ID NO: 20, CDR3β comprises or consists of the amino acid sequence SEQ ID NO: 50, and wherein the DNAJB1-PRKACA antigenic peptide comprises or consists of SEQ ID NO: 5; or
s) CDR3α comprises or consists of the amino acid sequence SEQ ID NO: 21, CDR3β comprises or consists of the amino acid sequence SEQ ID NO: 51, and wherein the DNAJB1-PRKACA antigenic peptide comprises or consists of SEQ ID NO: 5; or
t) CDR3α comprises or consists of the amino acid sequence SEQ ID NO: 21, CDR3β comprises or consists of the amino acid sequence SEQ ID NO: 63, and wherein the DNAJB1-PRKACA antigenic peptide comprises or consists of SEQ ID NO: 5; or
u) CDR3α comprises or consists of the amino acid sequence SEQ ID NO: 22, CDR3β comprises or consists of the amino acid sequence SEQ ID NO: 52, and wherein the DNAJB1-PRKACA antigenic peptide comprises or consists of SEQ ID NO: 5; or
v) CDR3α comprises or consists of the amino acid sequence SEQ ID NO: 23, CDR3β comprises or consists of the amino acid sequence SEQ ID NO: 53, and wherein the DNAJB1-PRKACA antigenic peptide comprises or consists of SEQ ID NO: 5; or
w) CDR3α comprises or consists of the amino acid sequence SEQ ID NO: 24, CDR3β comprises or consists of the amino acid sequence SEQ ID NO: 54, and wherein the DNAJB1-PRKACA antigenic peptide comprises or consists of SEQ ID NO: 5; or
x) CDR3α comprises or consists of the amino acid sequence SEQ ID NO: 25, CDR3β comprises or consists of the amino acid sequence SEQ ID NO: 55, and wherein the DNAJB1-PRKACA antigenic peptide comprises or consists of SEQ ID NO: 5; or
y) CDR3α comprises or consists of the amino acid sequence SEQ ID NO: 34, CDR3β comprises or consists of the amino acid sequence SEQ ID NO: 55, and wherein the DNAJB1-PRKACA antigenic peptide comprises or consists of SEQ ID NO: 5; or
z) CDR3α comprises or consists of the amino acid sequence SEQ ID NO: 26, CDR3β comprises or consists of the amino acid sequence SEQ ID NO: 56, and wherein the DNAJB1-PRKACA antigenic peptide comprises or consists of SEQ ID NO: 5; or
α) CDR3α comprises or consists of the amino acid sequence SEQ ID NO: 27, CDR3β comprises or consists of the amino acid sequence SEQ ID NO: 57, and wherein the DNAJB1-PRKACA antigenic peptide comprises or consists of SEQ ID NO: 5; or
β) CDR3α comprises or consists of the amino acid sequence SEQ ID NO: 28, CDR3β comprises or consists of the amino acid sequence SEQ ID NO: 58, and wherein the DNAJB1-PRKACA antigenic peptide comprises or consists of SEQ ID NO: 5; or
γ) CDR3α comprises or consists of the amino acid sequence SEQ ID NO: 29, CDR3β comprises or consists of the amino acid sequence SEQ ID NO: 59, and wherein the DNAJB1-PRKACA antigenic peptide comprises or consists of SEQ ID NO: 5; or
δ) CDR3α comprises or consists of the amino acid sequence SEQ ID NO: 30, CDR3β comprises or consists of the amino acid sequence SEQ ID NO: 60, and wherein the DNAJB1-PRKACA antigenic peptide comprises or consists of SEQ ID NO: 5; or
ε) CDR3α comprises or consists of the amino acid sequence SEQ ID NO: 31, CDR3β comprises or consists of the amino acid sequence SEQ ID NO: 61, and wherein the DNAJB1-PRKACA antigenic peptide comprises or consists of SEQ ID NO: 5;
and wherein said CDR sequences may comprise one or more amino acid mutation(s) selected from amino acid insertion, deletion and/or substitution,
wherein, preferably,
said antigen binding protein is a single chain TCR (scTCR) or a single-chain bispecific antibody, and/or
wherein the first polypeptide further comprises an α chain constant domain or a γ chain constant domain and the second polypeptide further comprises a β chain constant domain or a δ chain constant domain, and/or
wherein said first variable domain is part of a TCR α chain or a TCR γ chain and/or
wherein said second variable domain is part of a TCR β chain or a TCR δ chain, and/or
wherein the first variable domain is a TCR α variable domain, and the second variable domain is a TCR β variable domain, and/or
wherein said antigen binding protein comprises at least one amino acid mutation conferring increased stability, surface expression and/or pairing.

6. The antigen binding protein according to claim 5, further comprising one or more of the following:
(i) one or more further antigen binding sites;
(ii) a transmembrane region in said first polypeptide chain and/or said second polypeptide chain, optionally comprising a cytoplasmic signaling region;
(iii) a diagnostic agent;
(iv) a therapeutic agent; or
(v) a PK modifying moiety,
wherein, preferably,
said antigen binding protein is a therapeutic antigen binding protein and/or a diagnostic antigen binding protein.

7. An isolated nucleic acid molecule comprising a nucleotide sequence encoding the peptide or a variant thereof according to claim 1 or 2, the antibody or fragment thereof according to claim 3, the T-cell receptor according to claim 4, or antigen binding protein according to claim 5 or 6.

8. An isolated nucleic acid molecule comprising a nucleotide sequence selected from the group consisting of SEQ ID NO: 64 to 119.

9. A cloning or expression vector comprising the isolated nucleic acid molecule according to claim 7 or 8.

10. A cloning or expression vector encoding an antigen binding protein which specifically binds at least one DNAJB1-PRKACA antigenic peptide in complex with a major histocompatibility complex (MHC) molecule, wherein said vector comprises, under the control of a promoter, a first nucleotide sequence which encodes a first variable domain that comprises at least one complementary determining region (CDR), CDR3α, and a second nucleotide sequence which encodes a second variable domain that comprises at least one CDR, CDR3β, wherein
a) the first nucleotide sequence comprises or consists of SEQ ID NO: 64, the second nucleotide sequence comprises or consists of SEQ ID NO: 94, and wherein the DNAJB1-PRKACA antigenic peptide comprises or consists of SEQ ID NO: 1; or
b) the first nucleotide sequence comprises or consists of SEQ ID NO: 65, the second nucleotide sequence comprises or consists of SEQ ID NO: 95, and wherein the DNAJB1-PRKACA antigenic peptide comprises or consists of SEQ ID NO: 1; or
c) the first nucleotide sequence comprises or consists of SEQ ID NO: 66, the second nucleotide sequence comprises or consists of SEQ ID NO: 96, and wherein the DNAJB1-PRKACA antigenic peptide comprises or consists of SEQ ID NO: 1; or
d) the first nucleotide sequence comprises or consists of SEQ ID NO: 67, the second nucleotide sequence comprises or consists of SEQ ID NO: 97, and wherein the DNAJB1-PRKACA antigenic peptide comprises or consists of SEQ ID NO: 1; or
e) the first nucleotide sequence comprises or consists of SEQ ID NO: 68, the second nucleotide sequence comprises or consists of SEQ ID NO: 98, and wherein the DNAJB1-PRKACA antigenic peptide comprises or consists of SEQ ID NO: 1; or
f) the first nucleotide sequence comprises or consists of SEQ ID NO: 69, the second nucleotide sequence comprises or consists of SEQ ID NO: 99, and wherein the DNAJB1-PRKACA antigenic peptide comprises or consists of SEQ ID NO: 1; or
g) the first nucleotide sequence comprises or consists of SEQ ID NO: 70, the second nucleotide sequence comprises or consists of SEQ ID NO: 100, and wherein the DNAJB1-PRKACA antigenic peptide comprises or consists of SEQ ID NO: 1; or
h) the first nucleotide sequence comprises or consists of SEQ ID NO: 71, the second nucleotide sequence comprises or consists of SEQ ID NO: 101, and wherein the DNAJB1-PRKACA antigenic peptide comprises or consists of SEQ ID NO: 1; or
i) the first nucleotide sequence comprises or consists of SEQ ID NO: 72, the second nucleotide sequence comprises or consists of SEQ ID NO: 102, and wherein the DNAJB1-PRKACA antigenic peptide comprises or consists of SEQ ID NO: 1; or
j) the first nucleotide sequence comprises or consists of SEQ ID NO: 73, the second nucleotide sequence comprises or consists of SEQ ID NO: 103, and wherein the DNAJB1-PRKACA antigenic peptide comprises or consists of SEQ ID NO: 1; or
k) the first nucleotide sequence comprises or consists of SEQ ID NO: 90, the second nucleotide sequence comprises or consists of SEQ ID NO: 103, and wherein the DNAJB1-PRKACA antigenic peptide comprises or consists of SEQ ID NO: 1; or
l) the first nucleotide sequence comprises or consists of SEQ ID NO: 74, the second nucleotide sequence comprises or consists of SEQ ID NO: 104, and wherein the DNAJB1-PRKACA antigenic peptide comprises or consists of SEQ ID NO: 1; or
m) the first nucleotide sequence comprises or consists of SEQ ID NO: 75, the second nucleotide sequence comprises or consists of SEQ ID NO: 105, and wherein the DNAJB1-PRKACA antigenic peptide comprises or consists of SEQ ID NO: 1; or
n) the first nucleotide sequence comprises or consists of SEQ ID NO: 91, the second nucleotide sequence comprises or consists of SEQ ID NO: 105, and wherein the DNAJB1-PRKACA antigenic peptide comprises or consists of SEQ ID NO: 1; or
o) the first nucleotide sequence comprises or consists of SEQ ID NO: 76, the second nucleotide sequence comprises or consists of SEQ ID NO: 106, and wherein the DNAJB1-PRKACA antigenic peptide comprises or consists of SEQ ID NO: 1; or
p) the first nucleotide sequence comprises or consists of SEQ ID NO: 77, the second nucleotide sequence comprises or consists of SEQ ID NO: 107, and wherein the DNAJB1-PRKACA antigenic peptide comprises or consists of SEQ ID NO: 5; or
q) the first nucleotide sequence comprises or consists of SEQ ID NO: 77, the second nucleotide sequence comprises or consists of SEQ ID NO: 120, and wherein the DNAJB1-PRKACA antigenic peptide comprises or consists of SEQ ID NO: 5; or
r) the first nucleotide sequence comprises or consists of SEQ ID NO: 78, the second nucleotide sequence comprises or consists of SEQ ID NO: 108, and wherein the DNAJB1-PRKACA antigenic peptide comprises or consists of SEQ ID NO: 5; or
s) the first nucleotide sequence comprises or consists of SEQ ID NO: 79, the second nucleotide sequence comprises or consists of SEQ ID NO: 109, and wherein the DNAJB1-PRKACA antigenic peptide comprises or consists of SEQ ID NO: 5; or
t) the first nucleotide sequence comprises or consists of SEQ ID NO: 79, the second nucleotide sequence comprises or consists of SEQ ID NO: 121, and wherein the DNAJB1-PRKACA antigenic peptide comprises or consists of SEQ ID NO: 5; or
u) the first nucleotide sequence comprises or consists of SEQ ID NO: 80, the second nucleotide sequence comprises or consists of SEQ ID NO: 110, and wherein the DNAJB1-PRKACA antigenic peptide comprises or consists of SEQ ID NO: 5; or
v) the first nucleotide sequence comprises or consists of SEQ ID NO: 81, the second nucleotide sequence comprises or consists of SEQ ID NO: 111, and wherein the DNAJB1-PRKACA antigenic peptide comprises or consists of SEQ ID NO: 5; or
w) the first nucleotide sequence comprises or consists of SEQ ID NO: 82, the second nucleotide sequence comprises or consists of SEQ ID NO: 112, and wherein the DNAJB1-PRKACA antigenic peptide comprises or consists of SEQ ID NO: 5; or
x) the first nucleotide sequence comprises or consists of SEQ ID NO: 83, the second nucleotide sequence comprises or consists of SEQ ID NO: 113, and wherein the DNAJB1-PRKACA antigenic peptide comprises or consists of SEQ ID NO: 5; or
y) the first nucleotide sequence comprises or consists of SEQ ID NO: 84, the second nucleotide sequence comprises or consists of SEQ ID NO: 114, and wherein the DNAJB1-PRKACA antigenic peptide comprises or consists of SEQ ID NO: 5; or
z) the first nucleotide sequence comprises or consists of SEQ ID NO: 93, the second nucleotide sequence comprises or consists of SEQ ID NO: 114, and wherein the DNAJB1-PRKACA antigenic peptide comprises or consists of SEQ ID NO: 5; or
α) the first nucleotide sequence comprises or consists of SEQ ID NO: 85, the second nucleotide sequence comprises or consists of SEQ ID NO: 115, and wherein the DNAJB1-PRKACA antigenic peptide comprises or consists of SEQ ID NO: 5; or
β) the first nucleotide sequence comprises or consists of SEQ ID NO: 86, the second nucleotide sequence comprises or consists of SEQ ID NO: 116, and wherein the DNAJB1-PRKACA antigenic peptide comprises or consists of SEQ ID NO: 5; or
γ) the first nucleotide sequence comprises or consists of SEQ ID NO: 87, the second nucleotide sequence comprises or consists of SEQ ID NO: 117, and wherein the DNAJB1-PRKACA antigenic peptide comprises or consists of SEQ ID NO: 5; or
δ) the first nucleotide sequence comprises or consists of SEQ ID NO: 88, the second nucleotide sequence comprises or consists of SEQ ID NO: 118, and wherein the DNAJB1-PRKACA antigenic peptide comprises or consists of SEQ ID NO: 5; or
ε) the first nucleotide sequence comprises or consists of SEQ ID NO: 89, the second nucleotide sequence comprises or consists of SEQ ID NO: 119, and wherein the DNAJB1-PRKACA antigenic peptide comprises or consists of SEQ ID NO: 5;
and wherein said first and/or second nucleotide sequences may comprise one or more nucleic acid mutation(s) selected from nucleotide insertion, deletion and/or substitution.

11. A recombinant host cell comprising the peptide or a variant thereof according to claim 1 or 2, the antibody or fragment thereof according to claim 3, the T-cell receptor according to claim 4, the antigen binding protein according to claims 5 or 6, the isolated nucleic acid molecule according to claim 7 or 8, or the vector according to claim 9 or 10, wherein said host cell preferably is selected from a mammalian or human cell, further preferably from an antigen presenting cell, such as a dendritic cell, a T-cell or an NK cell.

12. An *in vitro* method for producing activated T cells, the method comprising contacting *in vitro* T cells with antigen loaded human class I or II MHC molecules expressed on the surface of a suitable antigen-presenting cell or an artificial construct mimicking an antigen-presenting cell for a period of time sufficient to activate said T cells in an antigen specific manner, wherein said antigen is a peptide or a variant thereof according to claim 1 or 2.

13. An activated T cell, produced by the method according to claim 12, that selectively recognizes a cell which presents a polypeptide comprising or consisting of the peptide or a variant thereof according to claim 1 or 2.

14. A pharmaceutical composition, preferably a vaccine, comprising at least one active ingredient selected from the group consisting of the peptide or a variant thereof according to claim 1 or 2, the antibody or fragment thereof according to claim 3, the T-cell receptor according to claim 4, the antigen binding protein according to any of the claims 5 to 8, the isolated nucleic acid molecule according to claim 9 or 10, or the vector according to claim 11 or 12, the recombinant host cell according to claim 13, or the activated T lymphocyte according to claim 15, or a conjugated or labelled active ingredient, and a pharmaceutically acceptable carrier, and optionally, pharmaceutically acceptable excipients and/or stabilizers.

15. A kit comprising:
(a) a container comprising a pharmaceutical composition containing the peptide or a variant thereof according to claim 1 or 2, the antibody or fragment thereof according to claim 3, the T-cell receptor according to claim 4, the antigen binding protein according to claim 5 or 6, the isolated nucleic acid molecule according to claim 7 or 8, or the vector according to claim 9 or 10, the recombinant host cell according to claim 11, or the activated T cell according to claim 13 in solution or in lyophilized form,
(b) optionally, a second container containing a diluent or reconstituting solution for the lyophilized formulation;
(c) optionally, at least one more peptide selected from the group consisting of SEQ ID NO: 1 to SEQ ID NO: 5, and
(d) optionally, instructions for (i) use of the solution or (ii) reconstitution and/or use of the lyophilized formulation.
